# EUROPEAN PATENT APPLICATION

(11) **EP 4 023 291 A1**
(43) Date of publication of application: **06.07.2022**
(21) Application number: 20858038.1
(22) Date of filing: 30.07.2020
(51) Int. Cl.: A61P 3/00, A61P 3/04, A61P 3/06, A61P 43/00, A23L 33/10, A23L 33/12, A61K 31/22, A61K 31/23

(54) **FAT METABOLISM PROMOTER**

(30) Priority: 30.08.2019 JP 2019158602; 30.04.2020 JP 2020080357
(71) Applicant: Asahi Group Holdings, Ltd., Tokyo 130-8602 (JP)
(72) Inventor: OHKI, Koji, Moriya-shi, Ibaraki 302-0106 (JP); KATO, Naoki, Moriya-shi, Ibaraki 302-0106 (JP); NAKAMURA, Fumiya, Moriya-shi, Ibaraki 302-0106 (JP); MIYAZAKI, Hidetoshi, Moriya-shi, Ibaraki 302-0106 (JP)
(74) Representative: Müller-Boré & Partner Patentanwälte PartG mbB
(86) International application number: PCT/JP2020/029309
(87) International publication number: WO 2021/039269

(57) **Abstract**

[Problem]

To provide a novel technology by which lipid metabolism can be promoted.

[Solution]

A lipid metabolism promoter which comprises, as an active ingredient, a compound represented by general formula (1) 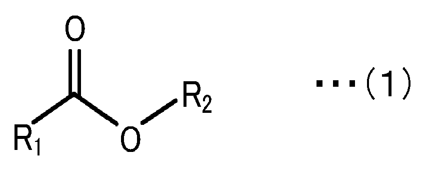 (in the formula, R1 and R2 independently represent an alkyl group having 1, 2, 3, 4, 5, 6 or 7 carbon atoms), wherein the compound represented by general formula (1) has an octanol/water partition coefficient represented by log Pow of 1.9-3.8 inclusive.

## Description

### Technical Field

The present invention relates to a lipid metabolism promoter comprising a compound represented by general formula (1): 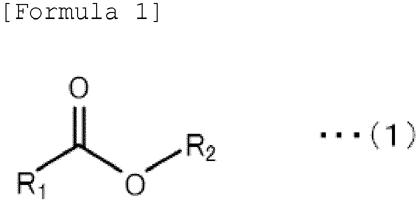 wherein R₁ and R₂ each independently represent an alkyl group having one, two, three, four, five, six, or seven carbon atoms.

### Background Art

By the global prevalence of the Western lifestyle, there are more and more opportunities to consume highcalorie and/or high-fat meals. In addition, lack of exercise is becoming a problem, and consumption of calories is decreasing as a trend. For these reasons, the population of obesity is more and more growing.

Obesity refers to condition involving excessive accumulation of fat, and one of causes for lifestyle diseases such as diabetes mellitus, hypertension, and dyslipidemia. In general, control of calorie intake by dietary restriction and control of calorie consumption by exercise are performed for preventing and improving obesity.

In recent years, techniques to prevent or improve obesity by promoting the metabolism of fat are under development. For example, Patent Literature 1 discloses a composition containing arginine, alanine, and phenylalanine as active ingredients, and discloses that lipid metabolism in individuals with obesity can be promoted by setting the contents of arginine, alanine, and phenylalanine in the composition to specific contents or more.

### Citation List

### Patent Literature

Patent Literature 1: JP 2019-099498A

### Summary of Invention

### Technical Problem

An object of the present invention is to provide a novel technique by which metabolism of fat can be promoted.

### Solution to Problem

The present inventors found that metabolism of fat can be promoted through administering of a compound having an octanol/water partition coefficient (log Pow) in the range of 1.9 or more and 3.8 or less and represented by general formula (1): 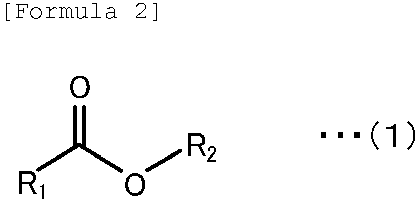 wherein R₁ and R₂ each independently represent an alkyl group having one, two, three, four, five, six, or seven carbon atoms. As a result, the present invention is completed.

The summary of the present invention is as follows.
[1] A lipid metabolism promoter comprising a compound represented by general formula (1): 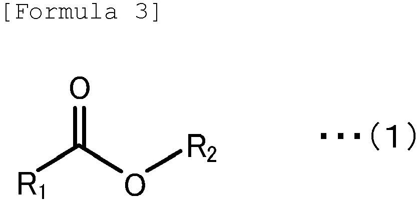 wherein R₁ and R₂ each independently represent an alkyl group having one, two, three, four, five, six, or seven carbon atoms,
   as an active ingredient, wherein the compound represented by general formula (1) has a log Pow indicating an octanol/water partition coefficient of 1.9 or more and 3.8 or less.
[2] The lipid metabolism promoter according to [1], wherein R₁ is one group selected from the group consisting of a methyl group, an ethyl group, a n-propyl group, a n-butyl group, a n-pentyl group, an isopentyl group, a n-hexyl group, and a n-heptyl group.
[3] The lipid metabolism promoter according to [1] or [2], wherein R₂ is one group selected from the group consisting of a methyl group, an ethyl group, a n-propyl group, a n-butyl group, a n-pentyl group, an isopentyl group, a 2-methylbutyl group, a n-hexyl group, and a n-heptyl group.
[4] The lipid metabolism promoter according to any one of [1] to [3], wherein the compound represented by general formula (1) is at least one compound selected from the group consisting of ethyl caproate, methyl caproate, propyl caproate, butyl caproate, pentyl caproate, ethyl caprylate, isoamyl acetate, isoamyl propionate, isoamyl butyrate, isoamyl caproate, pentyl acetate, heptyl acetate, methyl caprylate, ethyl 4-methylvalerate, 2-methylbutyl acetate, and ethyl valerate.
[5] The lipid metabolism promoter according to any one of [1] to [4], wherein the amount of the compound represented by general formula (1) to be ingested by an adult per day is 0.017 mg or more and 1.8 mg or less.
[6] The lipid metabolism promoter according to any one of [1] to [5], wherein the lipid metabolism promoter is a lipolysis promoter.
[7] The lipid metabolism promoter according to any one of [1] to [5], wherein the lipid metabolism promoter is a fat combustion promoter.
[8] A body heat production promoter comprising a compound represented by general formula (1):
   general formula (1): 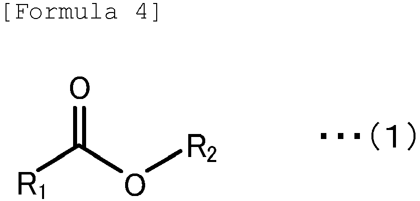 wherein R₁ and R₂ each independently represent an alkyl group having one, two, three, four, five, six, or seven carbon atoms,
   as an active ingredient, wherein the compound represented by general formula (1) has a log Pow indicating an octanol/water partition coefficient of 1.9 or more and 3.8 or less.
[9] Use of a compound represented by general formula (1): 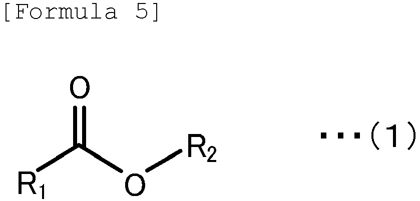 wherein R₁ and R₂ each independently represent an alkyl group having one, two, three, four, five, six, or seven carbon atoms, for promoting lipid metabolism.
[10] Use of a compound represented by general formula (1) : 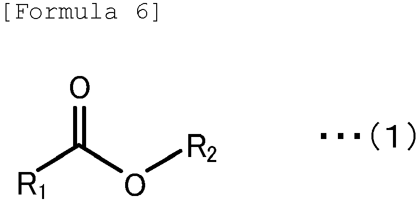 wherein R₁ and R₂ each independently represent an alkyl group having one, two, three, four, five, six, or seven carbon atoms, for producing a lipid metabolism promoter.
[11] Use of a compound represented by general formula (1) : 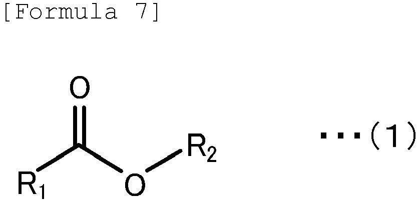 wherein R₁ and R₂ each independently represent an alkyl group having one, two, three, four, five, six, or seven carbon atoms, for promoting body heat production.
[12] Use of a compound represented by general formula (1) : 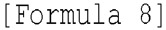 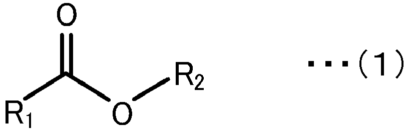 wherein R₁ and R₂ each independently represent an alkyl group having one, two, three, four, five, six, or seven carbon atoms, for producing a body heat production promoter.
[13] A therapeutic and/or preventive agent for at least one disease selected from the group consisting of dyslipidemia, hyperlipidemia, and obesity, the therapeutic and/or preventive agent comprising a compound represented by general formula (1): 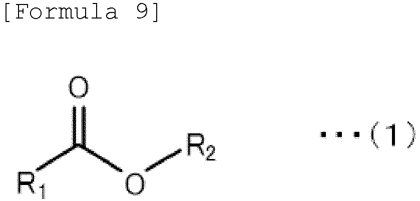 wherein R₁ and R₂ each independently represent an alkyl group having one, two, three, four, five, six, or seven carbon atoms.
[14] Use of a compound represented by general formula (1) : 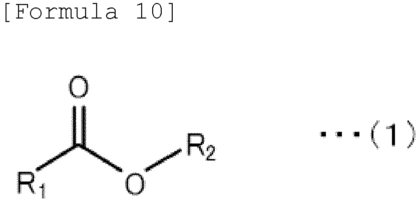 wherein R₁ and R₂ each independently represent an alkyl group having one, two, three, four, five, six, or seven carbon atoms, for treating and/or preventing at least one disease selected from the group consisting of dyslipidemia, hyperlipidemia, and obesity.
[15] Use of a compound represented by general formula (1) : 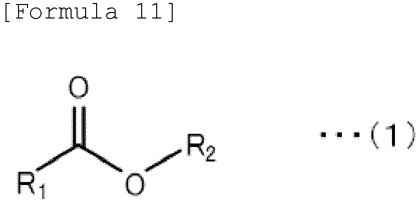 wherein R₁ and R₂ each independently represent an alkyl group having one, two, three, four, five, six, or seven carbon atoms, for producing a therapeutic and/or preventive agent for at least one disease selected from the group consisting of dyslipidemia, hyperlipidemia, and obesity.

### Advantageous Effects of Invention

The present invention can provide a novel technique by which metabolism of fat can be promoted.

### Brief Description of Drawings

[Figure 1] Figure 1 shows a diagram illustrating conditions for measurement of respiratory quotients (Test 1) .
[Figure 2] Figure 2 shows a graph representing the relationship between change rates of respiratory quotients and time (Test 1).
[Figure 3] Figure 3 shows a diagram illustrating conditions for measurement of body temperature (Test 2).
[Figure 4] Figure 4 shows a graph representing the relationship between interscapular skin temperature and time (Test 2).
[Figure 5] Figure 5 shows a graph representing the relationship between tympanic temperature and time (Test 2) .
[Figure 6] Figure 6 shows a diagram illustrating conditions for measurement of body temperature (Test 3).
[Figure 7] Figure 7 shows a graph representing the relationship between interscapular skin temperature and time (Test 3).
[Figure 8] Figure 8 shows a graph representing proportions of amounts of released glycerol (Test 4).
[Figure 9] Figure 9 shows a graph representing maximum values of amounts of change in RFU (ΔRFU) before and after addition of a promoter (Test 5).
[Figure 10] Figure 10 shows a graph representing the relationship between RFU and time (Test 5).
[Figure 11] Figure 11 shows a graph representing amounts of change in RFU (ΔRFU) after addition of an inhibitor relative to the RFU before addition of a promoter (Test 5) .
[Figure 12] Figure 12 shows a graph representing averages of respiratory quotients during resting (Test 6).
[Figure 13] Figure 13 shows a graph representing averages of lipid oxidation rates during resting (Test 6).
[Figure 14] Figure 14 shows a graph representing averages of respiratory quotients during resting and during exercising (Test 7).
[Figure 15] Figure 15 shows a graph representing averages of lipid oxidation rates during resting and during exercising (Test 7).
[Figure 16] Figure 16 shows a graph representing the relationship between interscapular skin temperature and time (Test 8).
[Figure 17] Figure 17 shows a graph representing the relationship between tympanic temperature and time (Test 8) .
[Figure 18] Figure 18 shows a graph representing maximum values of amounts of change in RFU (ΔRFU) before and after addition of a promoter (Test 9).

### Description of Embodiments

In description of group and atomic group in this specification, when substituted or unsubstituted is not explicitly indicated, both those without substituent and those with substituent(s) shall be included. For example, an "alkyl group" without specifying whether it is substituted or unsubstituted should be interpreted to refer to not only an alkyl group having no substituent (unsubstituted alkyl group) but also an alkyl group having a substituent (substituted alkyl group).

An embodiment of the present invention will be described bellow. The present embodiment relates to a lipid metabolism promoter that promotes metabolism of fat.

The lipid metabolism promoter of the present embodiment comprises a compound represented by general formula (1) (hereinafter, also referred to as "compound (1)"). Compound (1) comprised in the lipid metabolism promoter of the present embodiment has an octanol/water partition coefficient (log Pow) of 1.9 or more and 3.8 or less.

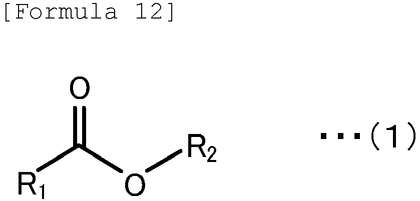

In the formula, R₁ and R₂ each independently represent an alkyl group having one, two, three, four, five, six, or seven carbon atoms.

Here, the phrase "R₁ and R₂ each independently" indicates that the alkyl groups (alkyl groups having one, two, three, four, five, six, or seven carbon atoms) constituting R₁ and R₂ may be identical or different.

Each alkyl group having one, two, three, four, five, six, or seven carbon atoms for R₁ and R₂ may be straight chain or branched chain. The alkyl groups having one, two, three, four, five, six, or seven carbon atoms for R₁ and R₂ may be cyclic, but are not needed to be cyclic. It is preferred for the alkyl groups having one, two, three, four, five, six, or seven carbon atoms for R₁ and R₂ are not cyclic. It is preferred for each of the alkyl groups having one, two, three, four, five, six, or seven carbon atoms for R₁ and R₂ to be an alkyl group having no substituent such as a hydroxy group and a benzyl group. Examples of the alkyl groups having one, two, three, four, five, six, or seven carbon atoms for R₁ and R₂ include a methyl group, an ethyl group, a n-propyl group, an isopropyl group (1-methylethyl group), a n-butyl group, a sec-butyl group (1-methylpropyl group), an isobutyl group (2-methylpropyl group), a tert-butyl group (1,1-dimethylethyl group), a n-pentyl group, an isopentyl group (3-methylbutyl group), a 2-methylbutyl group, a sec-pentyl group (1-methylbutyl group), a 3-pentyl group (1-ethylpropyl group), a tert-pentyl group (1,1-dimethylpropyl group), a n-hexyl group, an isohexyl group (4-methylpentyl group), a sec-hexyl group (1-methylpentyl group), a tert-hexyl group (1,1-dimethylbutyl group), a n-heptyl group, an isoheptyl group (5-methylhexyl group), a sec-heptyl group (1-methylhexyl group), and a tertheptyl group (1,1-dimethylhexyl group).

Preferred as the alkyl group having one, two, three, four, five, six, or seven carbon atoms for R₁ are a methyl group, an ethyl group, a n-propyl group, a n-butyl group, a n-pentyl group, an isopentyl group, a n-hexyl group, and a n-heptyl group, from the viewpoint of promoting metabolism of fat. Preferred as the alkyl group having one, two, three, four, five, six, or seven carbon atoms for R₂ are a methyl group, an ethyl group, a n-propyl group, a n-butyl group, a n-pentyl group, an isopentyl group, a 2-methylbutyl group, a n-hexyl group, and a n-heptyl group, from the viewpoint of promoting metabolism of fat. Preferred as a combination of alkyl groups for R₁ and R₂ if the alkyl group for R₁ is a methyl group, an ethyl group, a n-propyl group, a n-butyl group, a n-pentyl group, an isopentyl group, a n-hexyl group, or a n-heptyl group, are combinations with the alkyl group for R₂ being a methyl group, an ethyl group, a n-propyl group, a n-butyl group, a n-pentyl group, an isopentyl group, a 2-methylbutyl group, a n-hexyl group, or a n-heptyl group, from the viewpoint of promoting metabolism of fat.

The octanol/water partition coefficient, which is also designated as log Pow, is the logarithm of the ratio of concentrations of compound (1) in two phases (1-octanol phase and water phase) when compound (1) is added to two solvent phases composed of 1-octanol and water, and an equilibrium state is then reached. Pow is regarded as a physicochemical index indicating the hydrophobicity of a chemical, and in general, represented as a logarithmic value (log Pow). Smaller numerical values of log Pow indicate hydrophilicity, and larger numerical values of log Pow indicate lipophilicity.

Log Pow, which indicates an octanol/water partition coefficient, can be determined with a method described in JIS Z 7260-107 (2000). In addition, an octanol/water partition coefficient (log Pow) may be a value calculated with commercially available software or software on the Web using a quantitative structure-activity relationship algorithm. Examples of the quantitative structure-activity relationship algorithms include an XLOGP3 algorithm, which is an atom-based approach. XLOGP3 is described in known documents such as "Computation of Octanol-Water Partition Coefficients by Guiding an Additive Model with Knowledge.", Journal of Chemical Information and Modeling, 2007, Vol. 47, issue 6, pages 2140-2148. Examples of software capable of calculating octanol/water partition coefficients (log Pow) include XlogP (Shanghai Institute of Organic Chemistry, Chinese Academy of Sciences, available from http://WWW.sio-ccbg.ac.cn/software/xlogp3/). Moreover, results of calculation by XlogP are recorded in the database PubChem (http://pubchem.ncbi.nlm.nih.gov/#), and these can be utilized.

Examples of compound (1) include ethyl caproate (ethyl hexanoate), methyl caproate (methyl hexanoate), propyl caproate (propyl hexanoate), butyl caproate (butyl hexanoate), pentyl caproate (pentyl hexanoate), ethyl caprylate (ethyl octanoate), isoamyl acetate (3-methylbutyl acetate), isoamyl propionate (3-methylbutyl propanoate), isoamyl butyrate (3-methylbutyl butanoate), isoamyl caproate (3-methylbutyl hexanoate), pentyl acetate, heptyl acetate, methyl caprylate (methyl octanoate), ethyl 4-methylvalerate (ethyl 4-methylpentanoate), 2-methylbutyl acetate, and ethyl valerate (ethyl pentanoate). It is preferable from the viewpoint of promoting metabolism of fat that compound (1) be ethyl caproate, propyl caproate, butyl caproate, pentyl caproate, ethyl caprylate, isoamyl propionate, isoamyl butyrate, isoamyl caproate, pentyl acetate, heptyl acetate, or ethyl 4-methylvalerate, and it is more preferable that compound (1) be ethyl caproate or isoamyl acetate. For the lipid metabolism promoter of the present embodiment, two or more compounds may be used in combination as compound (1).

Ethyl caproate is a compound represented by formula (2):

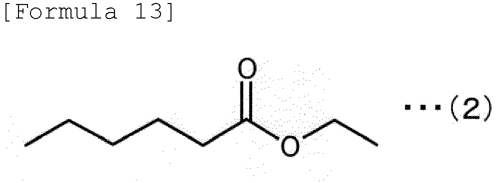

Isoamyl acetate is a compound represented by formula (3):

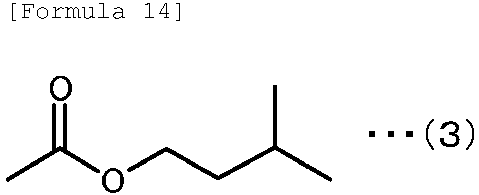

Compound (1) comprised in the lipid metabolism promoter of the present embodiment may be a compound biosynthesized by an organism, or a chemically synthesized (chemosynthesized) compound. Alternatively, a commercially available product may be used for compound (1) to be comprised in the lipid metabolism promoter of the present embodiment. For example, ethyl caproate and isoamyl acetate can be biosynthesized with a yeast. Biosynthesis of ethyl caproate with a yeast is described, for example, in JP2011-182745A, and biosynthesis of isoamyl acetate with a yeast is described, for example, in Japanese Patent JPH10-276767A.

The amount of compound (1) to be blended in the lipid metabolism promoter of the present embodiment is not limited, but such an amount that the amount of compound (1) to be ingested by an adult per day preferably reaches 0.0017 mg or more and 1.8 mg or less, and more preferably reaches 0.017 mg or more and 1.8 mg or less. The amount of ethyl caproate blended is even more preferably such an amount that the amount of compound (1) to be ingested by an adult per day reaches 0.017 mg or more and 0.17 mg or less, and the amount of isoamyl acetate blended is even more preferably such an amount that the amount of compound (1) to be ingested by an adult per day reaches 0.018 mg or more and 0.18 mg or less. The amount of compound (1) to be ingested by an adult per body weight (per day) is preferably 0.028 µg/kg or more and 30 µg/kg or less, and more preferably 0.28 µg/kg or more and 30 µg/kg or less. The amount of ethyl caproate to be ingested (per day) is even more preferably 0.28 µg/kg or more and 2.8 µg/kg or less, and the amount of isoamyl acetate to be ingested (per day) is even more preferably 0.30 µg/kg or more and 3.0 µg/kg or less. If the amount of compound (1) to be ingested per day is within the range described above, metabolism of fat can be more promoted than in the case that the amount of compound (1) to be ingested per day is out of the range described above. The frequency to ingest the lipid metabolism promoter of the present embodiment is not limited, and may be, for example, once per day.

The lipid metabolism promoter of the present embodiment has no limitation on the form of usage, and any form of usage may be employed such as a pharmaceutical, a quasi-drug, a food or beverage, and an additive. The lipid metabolism promoter of the present embodiment has no limitation on the state, and may be in any state such as liquid, solid, semisolid, and gel. An appropriate method may be employed, without limitation, for ingesting the lipid metabolism promoter of the present embodiment according to the form of usage and the state, and the lipid metabolism promoter of the present embodiment may be ingested, for example, orally.

In addition to compound (1), an additional component other than compound (1) may be comprised in the lipid metabolism promoter of the present embodiment. If the lipid metabolism promoter of the present embodiment is used for a pharmaceutical, a quasi-drug, or a food or beverage, for example, the lipid metabolism promoter of the present embodiment may comprise an excipient, a binder, a stabilizer, a disintegrator, a lubricant, a taste masking agent, a suspending agent, a coating agent, a solvent (e.g., ethanol), or a component comprised in a food or a beverage, in addition to compound (1).

In particular, if the lipid metabolism promoter of the present embodiment is used for a food or beverage, the food or beverage may be a normal food or beverage, or a food for special dietary uses such as a food for specified health uses, a food with nutrient function claims, or a food with functional claims. Examples of foods and beverages include: dietary supplements (supplements), milk, milk beverages, soft drinks (e.g., carbonated drinks, non-alcohol drinks, juices, coffee drinks, tea drinks, mineral waters, sports drinks), soybean drinks, probiotic drinks, cocoa, alcoholic drinks (e.g., beer, low-malt beer, other brewage, liqueur, sake, amazake, wine, shochu), processed milk, fermented milk, modified milk powder, drinks prepared with instant powder, and other drinks, cereals, yogurt, cheese, bread, pizza crust, ice cream, confectioneries including biscuits, crackers, candy, gummy candy, chewing gum, and others; liquid diets, foods for medical use, foods such as milk powder for infants, foods such as milk powder for nursing mothers, and feed stuffs.

The lipid metabolism promoter of the present embodiment has no limitation on the dosage form, and any dosage form may be appropriately employed according to the form of usage and state of the lipid metabolism promoter. If the lipid metabolism promoter of the present embodiment is used for a pharmaceutical, a quasi-drug, or a food or beverage such as a supplement, the dosage form may be, for example, a tablet, a pill, a capsule, a granule, a powder, a powdered agent, or a syrup.

Subjects to ingest the lipid metabolism promoter of the present embodiment are not limited to humans, and may be non-human animals. Examples of non-human animals include non-human higher vertebrates, in particular, non-human mammals, more specifically, pet animals such as dogs and cats, and domestic animals such as cattle, horses, pigs, and sheep. The subjects may be subjects under control of calorie intake by dietary restriction or the like, or subjects under control of calorie consumption by exercise or the like, but are not limited thereto.

Here, for promoting metabolism of fat, exercise may be performed after ingestion of the lipid metabolism promoter. An example of exercise is pedaling exercise using an AEROBIKE (R) as demonstrated later in Examples. In performing pedaling exercise, for example, the load may be 30 W or more, the rotational frequency may be 60 rpm or more, and the exercise time may be 30 minutes or longer. If exercise is performed after ingestion of the lipid metabolism promoter, for example, exercise may be initiated immediately after ingestion of the lipid metabolism promoter or within 2 hours after the ingestion.

Metabolism of fat can be promoted by ingesting the above-described lipid metabolism promoter of the present embodiment. Herein, metabolism of fat is a collective term for reactions that occur in the course of decomposition of fat into water and carbon dioxide. Metabolism of fat involves a reaction to decompose fat into free fatty acids and glycerol (hereinafter, also referred to as "lipolysis") and a reaction to decompose fatty acids into carbon dioxide and water (hereinafter, also referred to as "fat combustion"). Herein, the situation that metabolism of fat is promoted refers to such a situation that fat is more metabolized by ingesting the lipid metabolism promoter of the present embodiment than in the case that the lipid metabolism promoter of the present embodiment is not ingested. Ingestion of the lipid metabolism promoter of the present embodiment allows metabolism of fat to be promoted, and thus the reactions including lipolysis and fat combustion are promoted.

Although why the lipid metabolism promoter of the present embodiment promotes metabolism of fat has not been clarified, compound (1) is inferred to be able to pass through cell membranes with ease by virtue of the octanol/water partition coefficient (log Pow) being in the range of 1.9 or more and 3.8 or less. And compound (1) is inferred to be able to interact with the binding site of an ion channel present in cells, even when being decomposed by esterase in cells, by virtue of the number of carbon atoms of each of the alkyl groups represented by R₁ and R₂ in compound (1) being one, two, three, four, five, six, or seven. Accordingly, as demonstrated later in Examples, for example, the temperature-sensitive ion channel Transient Receptor Potential Ankyrin 1 (hereinafter, also referred to as "TRPA1"), which is expressed in sensory nerves, is inferred to be activated by compound (1), promoting metabolism of fat via neurotransmission. Another possible reason for the promotion of metabolism of fat is that compound (1) absorbed by the gastrointestinal tract acts on adipocytes (or fat) by virtue of its ability to pass through cell membranes with ease, thereby promoting metabolism of fat.

One of aspects of the lipid metabolism promoter of the present embodiment includes a lipid metabolism-promoting composition comprising compound (1) and any of the components other than compound (1) described above (hereinafter, also referred to as "additional component"). One of aspects of the lipid metabolism-promoting composition includes a lipid metabolism-promoting food or beverage composition comprising compound (1) and an additional component that can be comprised in a food or beverage (hereinafter, also referred to as "food or beverage component"). The lipid metabolism-promoting food or beverage composition may be used for a normal food or beverage, or for a food for special dietary uses such as a food for specified health uses, a food with nutrient function claims, or a food with functional claims. The lipid metabolism-promoting composition (or the lipid metabolism-promoting food or beverage composition) also can promote metabolism of fat as the lipid metabolism promoter of the present embodiment does.

The lipid metabolism promoter of the present embodiment may be used as an additive to be added to an additional component. An additional component to which the lipid metabolism promoter has been added as an additive can be used as a lipid metabolism-promoting composition capable of promoting metabolism of fat. In addition, the lipid metabolism promoter of the present embodiment can be used as an additive to be added to a food or beverage component. A food or beverage component to which the lipid metabolism promoter has been added as an additive can be used as a lipid metabolism-promoting food or beverage composition capable of promoting metabolism of fat.

Here, the lipid metabolism promoter of the present embodiment can promote a reaction to decompose fat into free fatty acids and glycerol (lipolysis). Accordingly, the lipid metabolism promoter of the present embodiment can be used as a lipolysis promoter capable of promoting lipolysis (i.e., applicable as a lipolysis promoter by the same configuration as the lipid metabolism promoter has). Herein, promoting lipolysis refers to such a situation that fat is more decomposed by ingesting the lipid metabolism promoter (or lipolysis promoter) of the present embodiment than in the case that the lipid metabolism promoter (or lipolysis promoter) of the present embodiment is not ingested.

One of aspects of the lipolysis promoter includes a lipolysis-promoting composition comprising compound (1) and an additional component other than compound (1). One of aspects of the lipolysis-promoting composition is a lipolysis-promoting food or beverage composition comprising compound (1) and an additional component that can be comprised in a food or beverage (food or beverage component). The lipolysis-promoting food or beverage composition may be used for a normal food or beverage, or for a food for special dietary uses such as a food for specified health uses, a food with nutrient function claims, or a food with functional claims. The lipolysis-promoting composition (or lipolysis-promoting food or beverage composition) can promote decomposition of fat as the lipolysis promoter does.

The lipolysis promoter of the present embodiment may be used as an additive to be added to an accessory component. An additional component to which the lipolysis promoter has been added as an additive can be used as a lipolysis-promoting composition capable of promoting lipolysis. In addition, the lipolysis promoter of the present embodiment can be used as an additive to be added to a food or beverage component. A food or beverage component to which the lipolysis promoter has been added as an additive can be used as a lipolysis-promoting food or beverage composition capable of promoting lipolysis.

The lipid metabolism promoter of the present embodiment can promote a reaction to decompose free fatty acids into carbon dioxide and water (fat combustion). Accordingly, the lipid metabolism promoter of the present embodiment can be used as a fat combustion promoter capable of promoting decomposition of free fatty acids (i.e., applicable as a fat combustion promoter by the same configuration as the lipid metabolism promoter has). Herein, promoting combustion of fat refers to such a situation that fat is more combusted by ingesting the lipid metabolism promoter (or fat combustion promoter) of the present embodiment than in the case that the lipid metabolism promoter (or fat combustion promoter) of the present embodiment is not ingested.

One of aspects of the fat combustion promoter is a fat-combustion-promoting composition comprising compound (1) and an additional component other than compound (1). One of aspects of the fat-combustion-promoting composition is a fat-combustion-promoting food or beverage composition comprising compound (1) and an additional component that is comprised in a food or beverage (food or beverage component). The fat-combustion-promoting food or beverage composition may be used for a normal food or beverage, or for a food for special dietary uses such as a food for specified health uses, a food with nutrient function claims, or a food with functional claims. The fat-combustion-promoting composition (fat-combustion-promoting food or beverage composition) can promote combustion of fat as the fat combustion promoter does.

The fat combustion promoter of the present embodiment may be used as an additive to be added to an additional component other than compound (1). An additional component to which the fat combustion promoter has been added as an additive can be used as a fat-combustion-promoting composition capable of promoting combustion of fat. In addition, the fat combustion promoter of the present embodiment can be used as an additive to be added to a food or beverage component. A food or beverage component to which the fat combustion promoter has been added as an additive can be used as a fat-combustion-promoting food or beverage composition capable of promoting combustion of fat.

In the above-described present embodiment, compound (1) is used for promoting lipid metabolism (including lipolysis and fat combustion), or for producing a lipid metabolism promoter (a lipolysis promoter or fat combustion promoter, too). Thus, the above-described present embodiment includes use of compound (1) for promoting lipid metabolism and use of compound (1) for producing a lipid metabolism promoter. Compound (1) to be used for promoting lipid metabolism and compound (1) to be used for producing a lipid metabolism promoter have the same configuration as compound (1) in the lipid metabolism promoter described above has, and hence detailed description thereof will be omitted.

As described above, the lipid metabolism promoter of the present embodiment can promote metabolism of fat (including lipolysis and fat combustion). Promoted metabolism of fat results in lowered blood triglyceride levels and LDL cholesterol levels, thus leading to treatment and/or prevention of various diseases in which blood triglyceride or LDL cholesterol are involved. For this reason, the lipid metabolism promoter of the present embodiment can be used for treating or preventing such a disease that the symptoms are ameliorated or the onset is prevented by promoting metabolism of fat. Examples of such diseases include dyslipidemia, hyperlipidemia, obesity, hypercholesterolemia, hypertriglyceridemia, arteriosclerosis, and metabolic syndrome. That is, the lipid metabolism promoter of the present embodiment can be used as a therapeutic and/or preventive agent for at least one disease selected from the group consisting of dyslipidemia, hyperlipidemia, obesity, hypercholesterolemia, hypertriglyceridemia, arteriosclerosis, and metabolic syndrome (applicable as a therapeutic and/or preventive agent for at least one disease selected from the group consisting of dyslipidemia, hyperlipidemia, obesity, hypercholesterolemia, hypertriglyceridemia, arteriosclerosis, and metabolic syndrome by the same configuration as the lipid metabolism promoter of the present embodiment has).

In the above-described embodiment relating to a therapeutic agent or a preventive agent, compound (1) is used for treating and/or preventing at least one disease selected from the group consisting of dyslipidemia, hyperlipidemia, obesity, hypercholesterolemia, hypertriglyceridemia, arteriosclerosis, and metabolic syndrome. Thus, the above-described embodiment relating to a therapeutic agent or a preventive agent includes use of compound (1) for treating and/or preventing at least one disease selected from the group consisting of dyslipidemia, hyperlipidemia, obesity, hypercholesterolemia, hypertriglyceridemia, arteriosclerosis, and metabolic syndrome. Compound (1) to be used for the above-described treatment and/or prevention has the same configuration as compound (1) in the lipid metabolism promoter described above has, and hence detailed description thereof will be omitted.

In the above-described embodiment relating to a therapeutic agent or a preventive agent, compound (1) is used for producing a therapeutic and/or preventive agent for at least one disease selected from the group consisting of dyslipidemia, hyperlipidemia, obesity, hypercholesterolemia, hypertriglyceridemia, arteriosclerosis, and metabolic syndrome. Thus, the above-described embodiment relating to a therapeutic agent or a preventive agent includes use of compound (1) for producing a therapeutic and/or preventive agent for at least one disease selected from the group consisting of dyslipidemia, hyperlipidemia, obesity, hypercholesterolemia, hypertriglyceridemia, arteriosclerosis, and metabolic syndrome. Compound (1) to be used for producing the above-described therapeutic and/or preventive agent has the same configuration as compound (1) in the lipid metabolism promoter described above has, and hence detailed description thereof will be omitted.

Here, the lipid metabolism promoter of the present embodiment can be used as a body heat production promoter capable of promoting production of body heat (i.e., applicable as a body heat production promoter by the same configuration as the lipid metabolism promoter has). Herein, promoting production of body heat refers to such a situation that body heat is more producted by ingesting the lipid metabolism promoter (or body heat production promoter) of the present embodiment than in the case that the lipid metabolism promoter (or body heat production promoter) of the present embodiment is not ingested.

Although why the lipid metabolism promoter (or body heat production promoter) of the present embodiment can promote production of body heat has not been clarified, production of body heat is inferred to be promoted by energy generated when free fatty acids resulting from decomposition of fat promoted, as described above, by ingesting the lipid metabolism promoter (or body heat production promoter) of the present embodiment are decomposed, for example, in brown adipocytes. In addition, the lipid metabolism promoter of the present embodiment can activate TRPA1. The activation of TRPA1 is known to promote production of body heat (Sawai S et al., "The effect of carbonated water on body temperature", 2012, Proceedings of Annual Meating of the Japan Society for Bioscience, Biotechnology, and Agrochemistry, Presentation No.: 3J15a01; Yukiko MASAMOTO et al., "Intragastric Administration of TRPV1, TRPV3, TRPM8, and TRPA1 Agonists Modulates Autonomic Thermoregulation in Different Manners in Mice", Bioscience, Biotechnology, and Biochemistry, 2009, Volume 73, Issue 5, Pages 1021-1027). Another possible reason for the promotion of production of body heat is that compound (1) absorbed by the gastrointestinal tract acts on adipocytes (or fat) to promote decomposition of free fatty acids, as a result promoting production of body heat.

Here, since production of body heat is promoted by ingesting the lipid metabolism promoter (or body heat production promoter) of the present embodiment, lowering of body temperature can be more reduced than in the case that the lipid metabolism promoter (or body heat production promoter) of the present embodiment is not ingested. Accordingly, the lipid metabolism promoter (or body heat production promoter) of the present embodiment can improve cold sensation. Herein, cold sensation refers to such a state that any of the limbs, waist, stomach, back, shoulders, and other sites is felt cold. There are various possible causes for cold sensation, and reduction of body heat production is believed to be one of the causes. In general, chronic feeling of cold sensation irrespective of the season or outside temperature is referred to as susceptibility to coldness. Examples of symptoms associated with susceptibility to coldness include shoulder stiffness, headache, swelling, sleep disruption, limb numbness, limb pain, frequent urination, and lumbar pain.

One of aspects of the body heat production promoter is a body heat production-promoting composition comprising compound (1) and an additional component other than compound (1). One of aspects of the body heat production-promoting composition is a body heat production-promoting food or beverage composition comprising compound (1) and an additional component that can be comprised in a food or beverage (food or beverage component). The body heat production-promoting food or beverage composition may be used for a normal food or beverage, or for a food for special dietary uses such as a food for specified health uses, a food with nutrient function claims, or a food with functional claims. The body heat production-promoting composition (or body heat production-promoting food or beverage composition) also can promote production of body heat as the body heat production promoter does.

The body heat production promoter of the present embodiment may be used as an additive to be added to an additional component other than compound (1). An additional component to which the body heat production promoter has been added as an additive can be used as a body heat production-promoting composition capable of promoting production of body heat. In addition, the body heat production promoter of the present embodiment can be used as an additive to be added to a food or beverage component. A food or beverage component to which the body heat production promoter has been added as an additive can be used as a body heat production-promoting food or beverage composition capable of promoting production of body heat.

In the above-described embodiment relating to a body heat production promoter, compound (1) is used for promoting body heat production, or for producing a body heat production promoter. Thus, the above-described embodiment relating to a body heat production promoter includes use of compound (1) for promoting body heat production and use of compound (1) for producing a body heat production promoter. Compound (1) to be used for promoting body heat production and compound (1) to be used for producing a body heat production promoter have the same configuration as compound (1) in the lipid metabolism promoter described above has, and hence detailed description thereof will be omitted.

### Examples

Hereinafter, the present invention will be more specifically described with reference to Examples; however, the present invention is not limited to these.

### [Test 1 (Respiratory Quotient Measurement Test - Water Basis)]

In the present test, measurement of respiratory quotients was carried out for the test. The respiratory quotient is the ratio of the amount of carbon dioxide released per unit time relative to the amount of oxygen consumed per unit time, and respiratory quotients were calculated by using an Aero Monitor (R) (manufactured by MINATO MEDICAL SCIENCE CO., LTD.) in the present test. The specific testing method of the present test and the results of the test are shown in the following. The present test was carried out as a crossover study.

### (Testing Method)

In the present test, each test beverage was ingested by three subjects (adult males (BMI (Body Mass Index): 18.5 kg/m² or higher and lower than 25 kg/m²)), and the respiratory quotients of each subject were measured. Used as test beverages were 200 mL of water (Comparative Example 1), and 200 mL of test solutions obtained by adding ethyl caproate into water to reach concentrations of 0.1 ppm, 1 ppm, and 10 ppm (Examples 1 to 3). The test beverages of Examples 1 to 3 give 0.017 mg, 0.17 mg, and 1.7 mg of ethyl caproate, respectively, as the amount of ethyl caproate to be ingested. The log Pow of ethyl caproate was 2.4 (calculated with the same method as described later in Test 9). In Tests 2 to 7 described later, description on the log Pow of ethyl caproate (2.4) is omitted.

Ethanol was used as the solvent for ethyl caproate for the test beverages of Examples 1 to 3, and a consistent ethanol concentration of 0.0999% was used for the test beverages of Examples 1 to 3 in all cases. Used as the raw materials of the test beverages: ethyl caproate, ethanol, and water, were "Ethyl Hexanoate ≥98%, FCC, Food Grade" (manufactured by Sigma-Aldrich Co. LLC), "Material Alcohol K for Alcoholic Beverages, Purity: 95.4%" (manufactured by Daiichi Alcohol Co., Ltd., Limited), and "Oishii Mizu Tennen-sui Fuji-san" (manufactured by ASAHI SOFT DRINKS CO., LTD.). These raw materials were used also for test beverages in Tests 2, 3, 6, 7, and 8 described later.

First, pre-measurement was carried out prior to measurement of respiratory quotients in the present test. In the pre-measurement, the subjects after being fasted for 12 hours from 21:00 on the previous day put on a mask for measurement of expired breath, and then measurement of respiratory quotients was initiated. After the initiation of measurement of respiratory quotients, each subject was made to sit on a chair to rest for 10 minutes. In 10 minutes of resting in a sitting position after the initiation of measurement of the respiratory quotient, the average of the respiratory quotient for 5 minutes from minute 6 to minute 10 was obtained as the baseline respiratory quotient. In the present test, measurement of respiratory quotients was carried out in a thermostatic/humidistatic experimental room set to a temperature of 23 ± 2°C and a humidity of 45 ± 5%. The respiratory rate during the measurement of respiratory quotients was 4 sec/respiration, and measurements shown below were performed with the same method.

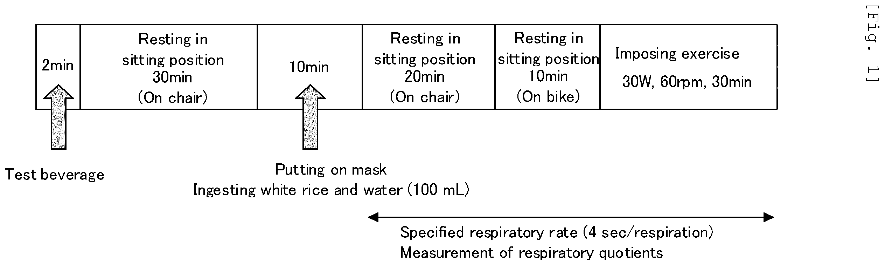

On day 1 of test, the subjects after being fasted for 12 hours from 21:00 on the previous day was subjected to measurement of the respiratory quotient under conditions shown in Figure 1. Specifically, the subjects was first made to ingest 200 mL of the test beverage of Comparative Example 1 in 2 minutes. After ingestion of the test beverage, each subject sat on a chair to rest for 30 minutes. Thereafter, each subject was made to ingest white rice (approximately 200 kcal) and 100 mL of water in 10 minutes, and put on a mask. After putting on a mask, measurement of respiratory quotients was initiated. For 20 minutes after the initiation of measurement of respiratory quotients, each subject was sitting on a chair to rest. After the lapse of 20 minutes, each subject sat on an AEROBIKE (R) (manufactured by Konami Sports Life Co., Ltd.) to rest for 10 minutes, and thereafter took exercise (30 W, 60 rpm) with the AEROBIKE (R) for 30 minutes. After the 30-minute exercise, the measurement of respiratory quotients was completed. Respiratory quotients were measured for 60 minutes in total.

From day 2 to day 4 of test, respiratory quotients were measured under the same conditions as on day 1 of test, except that the test beverages of Examples 1 to 3 were ingested. Different test beverages were ingested on different days of test. Specifically, the test subjects were made to ingest ethyl caproate with concentrations sequentially increased from day 2 to day 4 of test.

Data to be analyzed were as follows: in 20 minutes of resting in a sitting position from the initiation of measurement of respiratory quotients, respiratory quotients for 10 minutes from minute 11 to minute 20 were employed; in 30 minutes of exercise with the AEROBIKE (R), respiratory quotients for 20 minutes from minute 11 to minute 30 were employed. Averages of respiratory quotients employed were calculated at 1-minute intervals, and the change rates [%] thereof relative to the baseline respiratory quotient were determined. Each change rate [%] is a change rate as the baseline respiratory quotient is assumed as 100%.

### (Test Results)

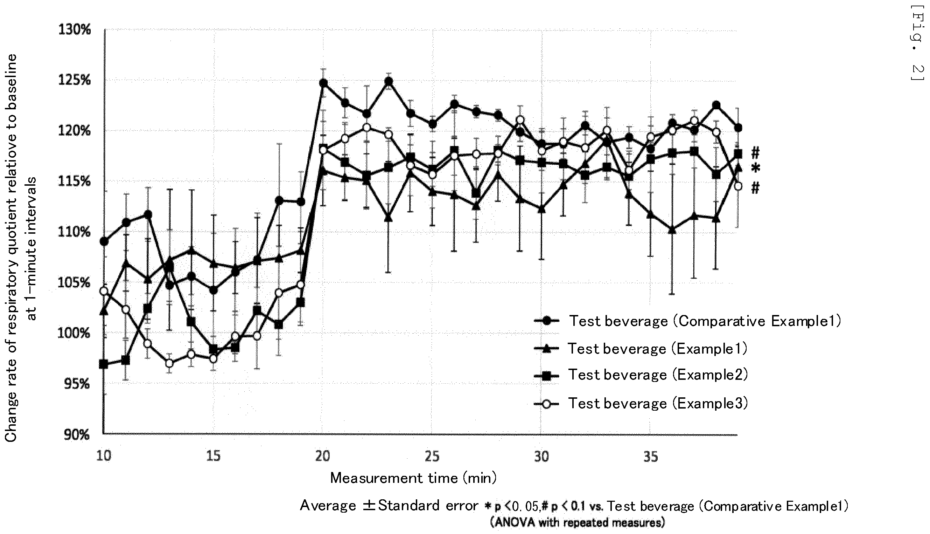

Figure 2 shows the results. In the present test, intergroup statistical significance test was carried out by analysis of variance (ANOVA) with repeated measures for averages of respiratory quotients at 1-minute intervals on each day of test with respect to change rates thereof to the baseline respiratory quotient.

As demonstrated in Figure 2, significant reduction of respiratory quotients was successfully found for the case with ingestion of the test beverage of Example 1 as compared with the case with ingestion of the test beverage of Comparative Example 1. Even for the case with ingestion of the test beverage of Example 2 and that with ingestion of the test beverage of Example 3, a tendency of reduced respiratory quotients was successfully found as compared with the case with ingestion of the test beverage of Comparative Example 1.

A reduced respiratory quotient indicates the increase in the amount of fat consumed. Therefore, the results of the present test confirmed that the test beverages of Examples 1 to 3 promoted both lipolysis and fat combustion, and thus promoted metabolism of fat.

### [Test 2 (Body Temperature Measurement Test - During Resting)]

In the present test, each of the subjects was made to ingest each test beverages, and their interscapular skin temperature and tympanic temperature (hereinafter in Test 2, these are also referred to as "body temperature", collectively) after ingestion were measured. The present test was carried out as a single-ingestion crossover study for 10 adult males and females (four males, six females) as subjects. The specific testing method of the present test and the results of the test are shown in the following.

In the present test, interscapular skin temperature was measured with a myBeat (R) WHS-2 (manufactured by UNION TOOL CO.), and tympanic temperature was measured with the ear thermometer CE Thermo 2 (manufactured by NIPRO CORPORATION).

### (Testing Method)

In the present test, 200 mL of water was used as a test beverage of Comparative Example 2. Used as a test beverage of Example 4 was 200 mL of a test solution obtained by adding ethyl caproate into water to reach a concentration of 1 ppm. Each subject was made to ingest different test beverages on day 1 and day 2 of test. The test beverage of Example 4 gives 0.17 mg of ethyl caproate as the amount of ethyl caproate to be ingested. Ethanol was used as the solvent for ethyl caproate for the test beverage of Example 4, and a consistent ethanol concentration of 0.0999% was used for the test beverage in all cases.

Before performing the present test, the subjects were forced to abstain from alcohol from the night before the test. On the day of the test, the subjects were forbidden from taking coffee or a highly spiced meal, made to finish lunch before 12:30, and forbidden from eating or drinking, except drinking water, after 12:30. All the subjects were made to wear the same specified short-sleeve shirt and long pants as clothes for the test.

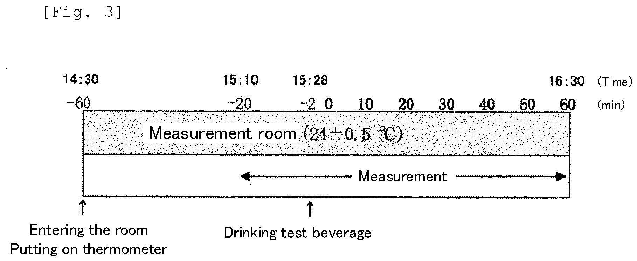

Measurement of body temperature was carried out under conditions shown in Figure 3, and the same conditions were applied on day 1 and day 2 of test. Specifically, the subjects were made to enter a measurement room with the room temperature set to 24 ± 0.5°C at 14:30, and conditioned to the environment for 40 minutes with a thermometer put on each subject. Subsequently, measurement of body temperature was initiated at 15:10, and the subjects were made to ingest 200 mL of a test beverage warmed to 37°C within 2 minutes at 15:28. Thereafter, the subjects were made to rest for 60 minutes with the measurement of body temperature continued, and the measurement of body temperature was completed at 16:30. In view of the diurnal variation of autonomic nerve activity, the measurements on day 1 and day 2 of test were carried out at the same time of day.

With the measurements at the timing immediately after drinking a test beverage, 15:30, defined as initial values (0-min data), average ± standard error at each time was calculated for each group on the basis of measurements at 10-minute intervals from the timing immediately after drinking a test beverage to the completion of measurement at 16:30 (measurements at min 10, 20, 30, 40, 50, and 60).

### (Test Results)

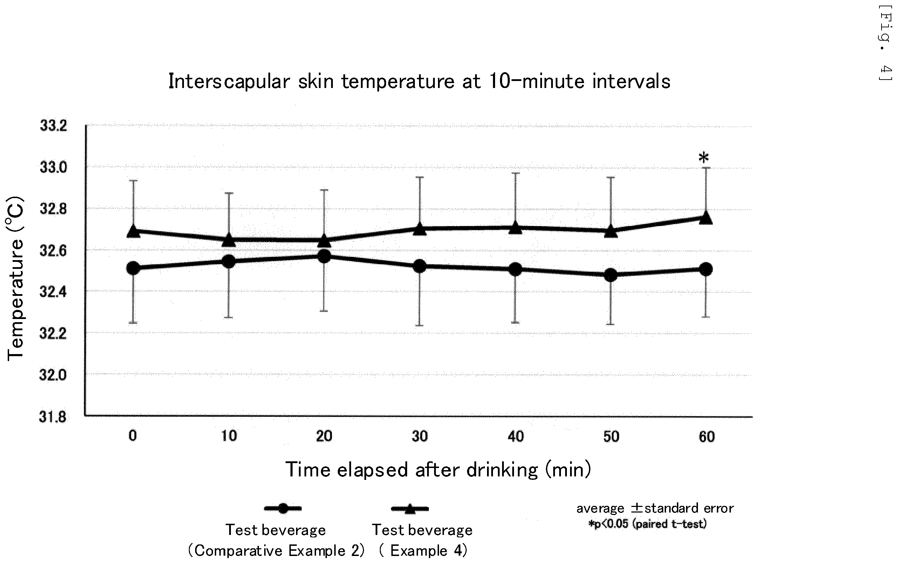

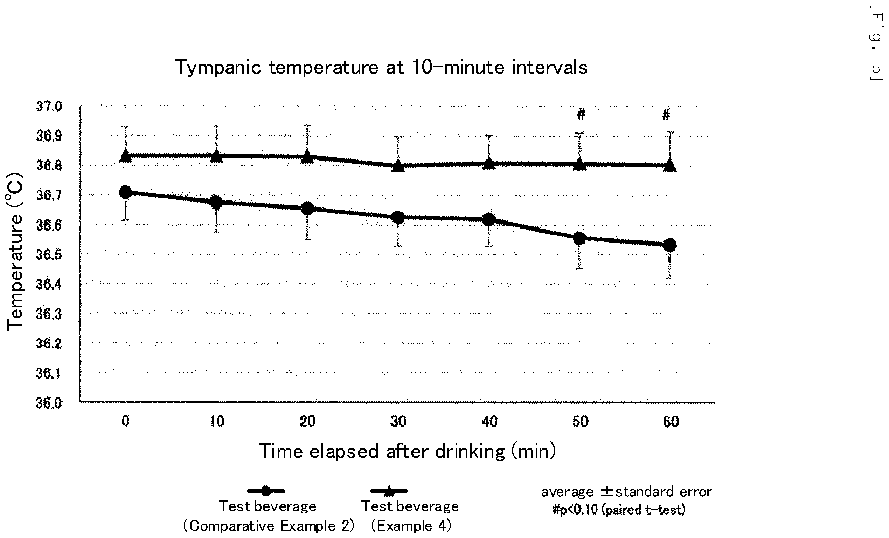

The results are shown in Table 1, Figure 4, Table 2, and Figure 5. Table 1 and Figure 4 show the results on interscapular skin temperature, and Table 2 and Figure 5 shows the results on tympanic temperature. In the present test, statistical significance test was carried out by paired t-test for differences between the groups at identical times. Significance levels of p < 0.05 were each determined to be a significant difference. In the present test, data analysis was performed with Excel (manufactured by Microsoft Corporation).

**[Table 1]**

| Interscapular skin temperature | | Elapsed time | | | | | | |
|---|---|---|---|---|---|---|---|---|
| | | 0 min | 10 min | 20 min | 30 min | 40 min | 50 min | 60 min |
| Test beverage (Comparative Example 2) | Average (°C) | 32.51 | 32.55 | 32.57 | 32.52 | 32.51 | 32.48 | 32.51 |
| | Standard error | 0.27 | 0.27 | 0.27 | 0.29 | 0.26 | 0.24 | 0.23 |
| Test beverage (Example 4) | Average (°C) | 32.69 | 32.65 | 32.65 | 32.71 | 32.71 | 32.70 | 32.76 |
| | Standard error | 0.24 | 0.22 | 0.24 | 0.25 | 0.26 | 0.26 | 0.24 |

**[Table 2]**

| Tympanic temperature | | Elapsed time | | | | | | |
|---|---|---|---|---|---|---|---|---|
| | | 0 min | 10 min | 20 min | 30 min | 40 min | 50 min | 60 min |
| Test beverage (Comparative Example 2) | Average (°C) | 36.71 | 36.68 | 36.66 | 36.63 | 36.62 | 36.56 | 36.53 |
| | Standard error | 0.13 | 0.14 | 0.14 | 0.14 | 0.15 | 0.15 | 0.15 |
| Test beverage (Example 4) | Average (°C) | 36.83 | 36.83 | 36.83 | 36.80 | 36.81 | 36.81 | 36.80 |
| | Standard error | 0.10 | 0.10 | 0.11 | 0.10 | 0.09 | 0.10 | 0.11 |

As demonstrated in Table 1 and Figure 4, an increasing tendency in interscapular skin temperature was found for the group with ingestion of the test beverage of Example 4, in particular, from minute 20 after drinking. By contrast, such an increasing tendency was not found for the group with ingestion of the test beverage of Comparative Example 2.

As demonstrated in Table 2 and Figure 5, a decreasing tendency in tympanic temperature was found for the group with ingestion of the test beverage of Comparative Example 2. By contrast, such a decreasing tendency was not found for the group with ingestion of the test beverage of Example 4.

Statistical test by intergroup comparison for the results shown in Figures 4 and 5 found no significant difference between the group with ingestion of the test beverage of Example 4 and the group with ingestion of the test beverage of Comparative Example 2 at minute 0, the timing immediately after drinking. On the other hand, for interscapular skin temperature shown in Figure 4, at minute 60 after drinking, the group with ingestion of the test beverage of Example 4 exhibited a significantly higher than the group with ingestion of the test beverage of Comparative Example 2 exhibited. For tympanic temperature shown in Figure 5, the group with ingestion of the test beverage of Example 4 was found to have a tendency of higher tympanic temperature than the group with ingestion of the test beverage of Comparative Example 2 exhibited at minute 50 and minute 60 after drinking. In particular, intragroup comparison for tympanic temperature shown in Figure 5 found that the tympanic temperature of the group with ingestion of the test beverage of Comparative Example 2 at minute 60 was significantly lower than that at minute 0. By contrast, no such result was found for the group with ingestion of the test beverage of Example 4.

Increase in interscapular skin temperature and reduction in lowering of tympanic temperature indicate promoted production of body heat. In particular, tympanic temperature is body temperature reflecting core body temperature, and hence reduction in lowering of tympanic temperature indicates that production of body heat is also promoted in the inside of a body. Since the interscapular part is in the vicinity of the brown adipose tissue, increase in interscapular skin temperature indicates that fatty acids are consumed in the brown adipose tissue and production of body heat is promoted. Therefore, the results of the present test confirmed that the test beverage of Example 4 promoted combustion of fat and further promoted production of body heat.

### [Test 3 (Body Temperature Measurement Test - During Exercising)]

In the present test, each of the subjects was made to ingest each test beverage, and their interscapular skin temperature during imposing exercise was measured. The present test was carried out for four adult males as subjects. Interscapular skin temperature was measured with a myBeat (R) WHS-2. The specific testing method of the present test and the results of the test are shown in the following.

### (Testing Method)

In the present test, a sweet-and-sour solution of less than 5 kcal/100 mL consisting of aspartame, acesulfame K, sucralose, anhydrous citric acid, trisodium citrate, and water was used as a test beverage of Comparative Example 3. A test solution obtained by adding ethyl caproate into the sweet-and-sour solution described above to reach a concentration of 1 ppm was used as a test beverage of Example 5. Each subject was made to ingest the test beverage of Comparative Example 3 on day 1 of test and to ingest the test beverage of Example 5 on day 2 of test. The test beverage of Example 5 gives 0.17 mg of ethyl caproate as the amount of ethyl caproate to be ingested. Ethanol was used as the solvent for ethyl caproate for the test beverage of Example 5, and a consistent ethanol concentration of 0.0999% was used for the test beverage in all cases.

Before performing the present test, the subjects were forced to abstain from alcohol from the night before the test. All the subjects were made to wear the same specified short-sleeve shirt and long pants as clothes for the test.

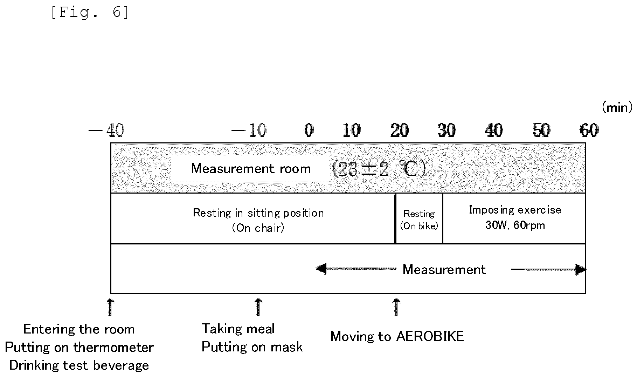

The subjects after being fasted from 21:00 on the previous day were subjected to measurement of interscapular skin temperature under conditions shown in Figure 6. The same conditions were applied on day 1 and day 2 of test. Specifically, first, the subjects were each made to enter a thermostatic/humidistatic experimental room set to a temperature of 23 ± 2°C and a humidity of 45 ± 5% at 8:30, and made to ingest a test beverage and sit on a chair. After resting by sitting a chair for 30 minutes, each of the subjects was made to ingest white rice (approximately 200 kcal) and 100 mL of water in 10 minutes, and put on a mask for measurement of expired breath. After putting on a mask, measurement of interscapular skin temperature was initiated. After resting by sitting on a chair for 20 minutes after the initiation of measurement of interscapular skin temperature, each subject was made to sit on an AEROBIKE (R) to further rest for 10 minutes. Thereafter, each subject was made to exercise with the AEROBIKE (R) for 30 minutes (30 W, 60 rpm), and the measurement of interscapular skin temperature was completed. In view of the diurnal variation of autonomic nerve activity, the measurements on day 1 and day 2 of test were carried out at the same time of day.

From measurements of interscapular skin temperature at the timing immediately after the initiation of exercise and those at 10-minute intervals after the initiation of exercise (measurements at minute 10, 20, and 30), average ± standard error at each time was calculated for each group.

### (Test Results)

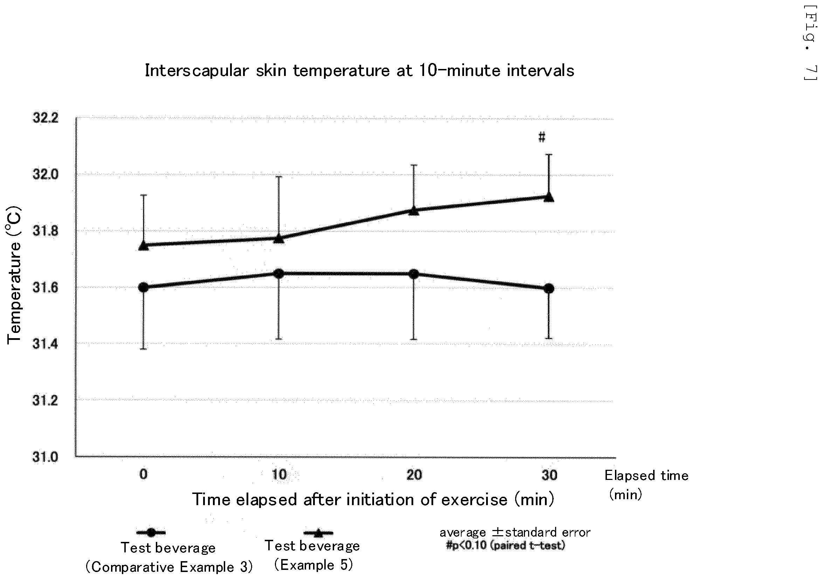

The results are shown in Table 3 and Figure 7. In the present test, statistical significance test was carried out by paired t-test for differences between the groups at identical times. Significance levels of p < 0.05 were each determined to be a significant difference. Data analysis was performed with Excel (manufactured by Microsoft Corporation).

**[Table 3]**

| Interscapular skin temperature | | Elapsed time | | | |
|---|---|---|---|---|---|
| | | 0 min | 10 min | 20 min | 30 min |
| Test beverage (Comparative Example 3) | Average (°C) | 31.60 | 31.65 | 31.65 | 31.60 |
| | Standard error | 0.22 | 0.23 | 0.23 | 0.18 |
| Test beverage (Example 5) | Average (°C) | 31.75 | 31.78 | 31.88 | 31.93 |
| | Standard error | 0.18 | 0.22 | 0.16 | 0.15 |

As demonstrated in Table 3 and Figure 7, the group with ingestion of the test beverage of Example 5 was successfully found to exhibit high interscapular skin temperature for a longer period than the group with ingestion of the test beverage of Comparative Example 3 exhibited. In particular, statistical test by intergroup comparison found that the group with ingestion of the test beverage of Example 5 had a tendency of higher interscapular skin temperature than the group with ingestion of the test beverage of Comparative Example 3 exhibited at minute 30 after the initiation of exercise (i.e., at minute 60 after the initiation of temperature measurement).

As described above, increase in interscapular skin temperature indicates that fatty acids are consumed in the brown adipose tissue and generation of body heat is promoted. Therefore, the results of the present test confirmed that the test beverage of Example 5 promoted combustion of fat also through exercise and further promoted generation of body heat.

### [Test 4 (Lipolysis Test)]

In the present test, the lipolysis-promoting effect of the promoter of the present embodiment was tested by quantifying glycerol released into culture supernatant through decomposition of fat accumulated in adipocytes. Mouse embryonic 3T3-L1 cells, which are progenitor cells of adipocytes and were used in the present test, were induced to differentiate to form adipocytes. The present test was carried out in accordance with a method described in "Effects of Quercetin Glucosides on Dietinduced Obesity in Mice - The Lipolytic Activity of Quercetin -" (Tateishi et al., Japanese Pharmacology & Therapeutics 2009; 37(2): 123-131). The specific testing method of the present test and the results of the test are shown in the following.

### (Testing Method)

In the present test, first, 3T3-L1 cells were suspended in Dulbecco's Modified Eagle medium (manufactured by FUJIFILM Wako Pure Chemical Corporation) (hereinafter, also referred to as "DMEM medium") to which penicillin-streptomycin (manufactured by Thermo Fisher Scientific, Inc.) and fetal bovine serum (manufactured by Sigma-Aldrich Co. LLC) had been added to reach concentrations of 100 Unit/mL, 100 µg/mL, and 10%, respectively, and seeded in a 12-well plate.

The 3T3-L1 cells were cultured under conditions of 5% CO₂ and 37°C until the cells became confluent (a state in which cells are totally covering the surface of a culture vessel). Two days after becoming confluent, the medium was exchanged with a medium obtained by adding dexamethasone (manufactured by NACALAI TESQUE, INC.), 3-isobutyl-1-methylxanthine (manufactured by NACALAI TESQUE, INC.), and insulin (manufactured by NACALAI TESQUE, INC.) to DMEM medium to reach concentrations of 0.25 µM, 0.5 mM, and 10 µg/mL, respectively. Thereafter, the 3T3-L1 cells were cultured at 5% CO₂ and 37°C for 2 days to induce the cells to differentiate into adipocytes.

After the induction of differentiation, medium exchange with a medium obtained by adding insulin to DMEM medium to reach a concentration of 5 µg/mL was performed every 2 or 3 days, and differentiation was maintained by culturing at 5% CO₂ and 37°C for 12 days in total. Thereafter, a medium is prepared as a test medium by adding penicillin-streptomycin (manufactured by Thermo Fisher Scientific, Inc.) and fetal bovine serum (manufactured by Sigma-Aldrich Co. LLC) to DMEM medium free of phenol red (1.0 g/L glucose) (manufactured by NACALAI TESQUE, INC.) to reach concentrations of 100 Unit/mL, 100 µg/mL, and 10%, respectively, and components shown in Table 4 were added to the test medium to reach concentrations shown in Table 4. The medium for the 3T3-L1 cells after induction of differentiation was exchanged with a medium to which components of any of Comparative Examples 4, 5 or Examples 6 to 8 had been added, and the 3T3-L1 cells were cultured at 5% CO₂ and 37°C for 2 days.

**[Table 4]**

| | Comparative Example 4 | Comparative Example 5 | Example 6 | Example 7 | Example 8 |
|---|---|---|---|---|---|
| Ethanol | 0.1% | 0.1% | 0.1% | 0.1% | 0.1% |
| Noradrenaline | - | 0.1 *_{µ}* M | 0.1 *_{µ}* M | 0.1 *_{µ}* M | 0.1 *_{µ}* M |
| Ethyl caproate | - | - | 0.1 ppm | 1 ppm | 10 ppm |

Ethyl caproate manufactured by Tokyo Chemical Industry Co., Ltd. was used for the ethyl caproate, ethanol manufactured by FUJIFILM Wako Pure Chemical Corporation was used for the ethanol, and noradrenaline manufactured by NACALAI TESQUE, INC. was used for the noradrenaline.

After culturing for 2 days, the culture supernatant was collected, and glycerol released into the culture supernatant was quantified with a Glycerol Assay kit (Cell-Based) (manufactured by Abcam). The ratio of the amount of glycerol in each medium to which the components of any of Comparative Example 5 and Examples 6 to 8 had been added relative to the amount of glycerol in the medium to which the component of Comparative Example 4 had been added (hereinafter, referred to as "control ratio") was determined to obtain the average ± standard error.

### (Test Results)

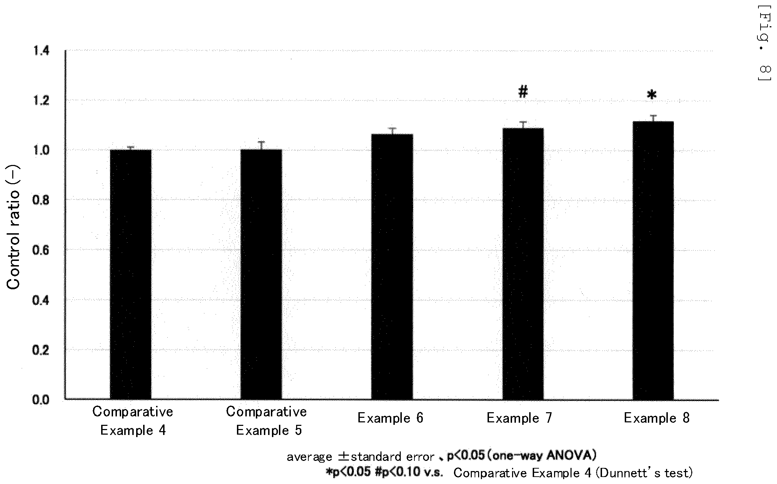

The results are shown in Table 5 and Figure 8. In the present test, statistical test was carried out by multiple comparison test with one-way analysis of variance (one-way ANOVA) followed by Dunnett's test. Significance levels of p < 0.05 were each determined to be a significant difference. These analyses were all performed with SPSS version 23 (manufactured by SPSS).

**[Table 5]**

| | Average | Standard error |
|---|---|---|
| Comparative Example 4 | 1.000 | 0.012 |
| Comparative Example 5 | 1.002 | 0.031 |
| Example 6 | 1.063 | 0.024 |
| Example 7 | 1.089 | 0.026 |
| Example 8 | 1.118 | 0.024 |

As demonstrated in Table 5 and Figure 8, almost no difference in the amount of released glycerol was found between the supernatant of the medium to which the component of Comparative Example 4 had been added and that to which the components of Comparative Example 5 had been added. On the other hand, more glycerol was released in the supernatant of each medium to which the components of any of Examples 6 to 8 had been added than in the supernatant of the medium to which the component of Comparative Example 4 had been added.

One-way ANOVA in the statistical test found significant differences among the groups, and hence intergroup multiple comparison test was carried out with Dunnett's test for Examples 6 to 8 against Comparative Example 4. The results found a significant increase in the amount of released glycerol for Example 8, in which the concentration of added ethyl caproate was 10 ppm, and an increasing tendency in the amount of released glycerol for Example 7, in which the concentration of added ethyl caproate was 1.0 ppm.

The results of the present test confirmed that decomposition of fat was promoted in the medium to which the components of any of Examples 6 to 8 had been added.

### [Test 5 (TRPA1 Activity Test)]

In the present test, the contribution of the lipid metabolism promoter of the present embodiment to TRPA1 activity was examined by checking the activation of the temperature-sensitive ion channel TRPA1 with a calcium imaging method. The specific testing method of the present test and the results of the test are shown in the following.

### (First Testing Method)

In the present test, first, human embryonic HEK293T cells were first suspended in DMEM medium to which penicillin-streptomycin (manufactured by Thermo Fisher Scientific, Inc.) and fetal bovine serum (manufactured by Sigma-Aldrich Co. LLC) were added to reach concentrations of 100 Unit/mL, 100 µg/mL, and 10%, respectively, seeded in a 96-well plate at a volume of 100 µL/well, and cultured under conditions of 5% CO₂ and 37°C until a cell density reached 70 to 90%.

Thereafter, the DMEM medium in each well was completely removed, to which OptiMEM medium (manufactured by Thermo Fisher Scientific, Inc.) containing the gene transfer reagent Lipofectamine 2000 (manufactured by Thermo Fisher Scientific, Inc.) and a hTRPA1 gene expression plasmid was added at a volume of 50 µL/well. Then, culture was performed under conditions of 37°C and 5% CO₂ for approximately 24 hours to transfer the TRPA1 gene into the HEK293T cells. The contents of Lipofectamine 2000 and the hTRPA1 gene expression plasmid per 50 µL of the OptiMEM medium were 0.3 µL and 400 ng, respectively. The TRPA1 gene can be confirmed with a DNA database such as GenBank.

When the density of the HEK293T cells transfected in the described manner (hereinafter, also referred to as "cells with forced expression of TRPA1") reached 90 to 100%, FLIPR Calcium 6 Assay Kit (manufactured by Molecular Devices, LLC.), a calcium-sensitive fluorescent dye, was added at a volume of 50 µL/well, and culture was performed under conditions of 37°C and 5% CO₂ for 2 hours. Here, the expression of TRPA1 on cell membranes was confirmed by response to cinnamaldehyde (manufactured by Wako Pure Chemical Industries, Ltd.), which is a known TRPA1 ligand. DMSO was used for dilution of cinnamaldehyde, and the DMSO concentration in each well was set to 0.1% in view of cytotoxicity.

Ethyl caproate was added to HEPES buffer (10 mM HEPES, 130 mM NaCl, 5 mM KCl, 10 mM glucose, 2 mM CaCl₂·2H₂O, 1.2 mM MgCl₂·6H₂O, pH = 7.40), and thus a test solution of Example 9 containing ethyl caproate in a concentration of 255 ppm was obtained. For a negative control (N.C.), HEPES buffer was used as a test solution of Comparative Example 6. Measurement of fluorescence intensity for the cells with forced expression of TRPA1 was initiated, and, 20 seconds thereafter, 50-µL portions of each of the test solutions prepared were added to three wells. After the lapse of a predetermined period of time, the measurement of fluorescence intensity was completed, and ratios of fluorescence intensity at each time after addition of a test solution as the average of fluorescence intensity before addition of a test solution was assumed as 1 (hereinafter, also referred to as "RFU (relative fluorescence units)") were determined from measurements of fluorescence intensity. Further, change rates in RFU before and after addition of a test solution (hereinafter, also referred to as "ΔRFU") were determined at each time after addition of a test solution. Fluorescence intensity was measured by using a FlexStation 3 (manufactured by Molecular Devices, LLC.). The measurement of fluorescence intensity was performed at room temperature, and each test solution was used for adding to wells at room temperature, too.

Also for HEK293T cells without transfer of the TRPA1 gene (hereinafter, also referred to as "non-transformed cells"), as a reference, 50 µL of the test solution of Example 9 and 50 µL of the test solution of Comparative Example 6 were added with the same method as performed for the cells with forced expression of TRPA1, and ΔRFU was determined.

It should be noted that the total volume of the OptiMEM medium and the FLIPR Calcium 6 Assay Kit contained in each well immediately before addition of a test solution was 100 µL, to which 50 µL of a test solution was then added, and hence ethyl caproate comprised in the test solution of Example 9 was 3-fold diluted in each well. Specifically, the concentration of ethyl caproate in each well with addition of the test solution of Example 9 was 100 ppm.

### (Results of First Test)

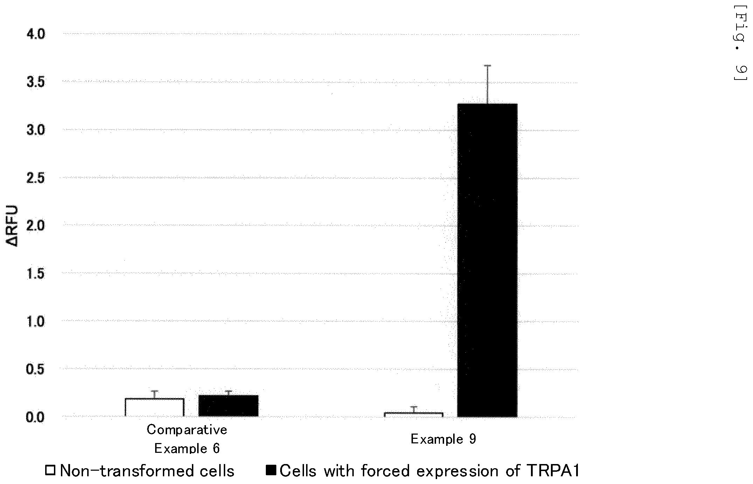

Table 6 and Figure 9 shows average ± standard deviation for maximum values of ΔRFU after addition of a test solution. In the present test, SoftMaxPro Vr. 6 (manufactured by Molecular Devices, LLC.) and Excel (manufactured by Microsoft Corporation) were used for data analysis.

**[Table 6]**

| | | Comparative Example 6 | Example 9 |
|---|---|---|---|
| Non-transformed cells | Average (Δ RFU) | 0.185 | 0.046 |
| | Standard deviation | 0.080 | 0.065 |
| Cells with forced expression of TRPA1 | Average (ΔRFU) | 0.217 | 3.275 |
| | Standard deviation | 0.050 | 0.400 |

As demonstrated in Table 6 and Figure 9, the test solution of Comparative Example 6 exhibited a maximum value of ΔRFU of about 0.2 for the cells with forced expression of TRPA1, and thus did not evoke cellular response. On the other hand, the test solution of Example 9 exhibited a maximum value of ΔRFU of about 3.3 for the cells with forced expression of TRPA1, and thus was successfully found to have evoked cellular response. From this result, the test solution of Example 9 was confirmed to have activated TRPA1.

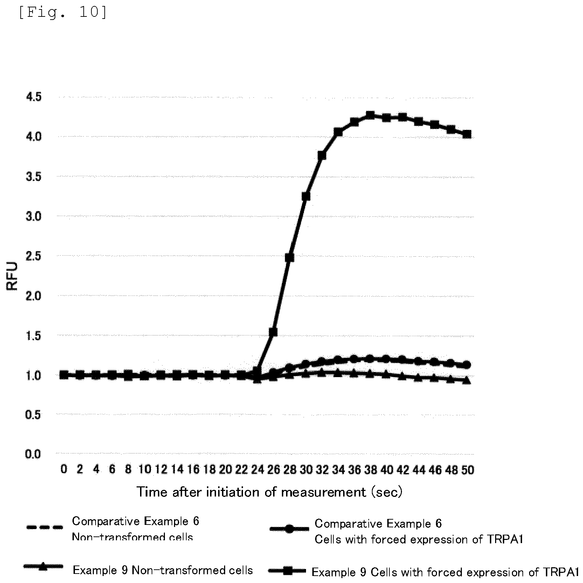

Figure 10 shows temporal variations of RFU before and after addition of each test solution. The maximum values of ΔRFU shown in Table 6 and Figure 9 are values determined on the basis of RFU shown in Figure 10. As can be understood from the results shown in Figure 10, the test solution of Example 9 evoked cellular response for the cells with forced expression of TRPA1, whereas the test solution of Comparative Example 6 did not evoke cellular response for the cells with forced expression of TRPA1.

### (Second Testing Method)

In the present test, the TRPA1 inhibitor HC03001 (manufactured by Sigma-Aldrich Co. LLC) was further added to the wells to which the test solution of Example 9 had been added in First Testing Method (co-addition). Subsequently, the fluorescence intensity in each well was confirmed to have become constant, and RFU was then determined (hereinafter, also referred to as "RFU after addition of the inhibitor"). The amount of change in RFU after addition of the inhibitor from RFU before addition of the test solution, which was assumed to be 1, was determined (hereinafter, also referred to as "ΔRFU after addition of the test solution"). DMSO was used for dilution of HC03001, and the DMSO concentration in each well was set to 0.1% in view of cytotoxicity.

### (Results of Second Test)

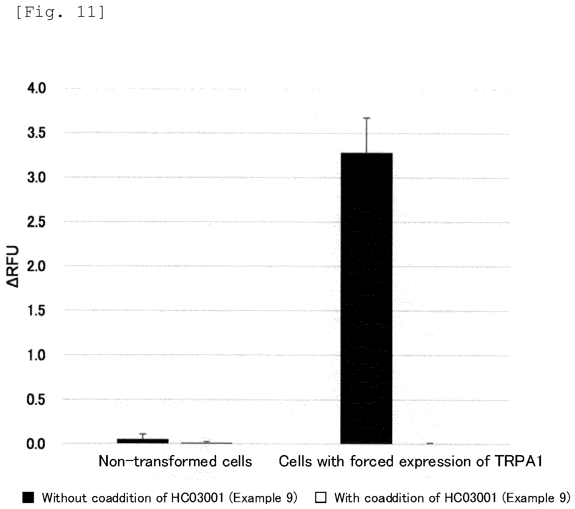

Figure 11 shows average ± standard deviation of ΔRFU after addition of the inhibitor (with coaddition of HC03001 in Figure 11). In Figure 11, maximum value ± standard deviation of ΔRFU before coaddition of the inhibitor is shown as a reference (without coaddition of HC03001 in Figure 11). SoftMax Pro Vr. 6 and Excel were used for data analysis as in Results of First Test.

As demonstrated in Figure 11, the cellular response of the cells with forced expression of TRPA1, the response evoked by addition of the test solution of Example 9, disappeared with addition of the TRPA1 inhibitor HC03001.

Results of First and Second Tests described above confirmed that the test solution of Example 9 activates TRPA1.

### [Test 6 (Respiratory Quotient Measurement Test - Sweet-and-Sour Solution Basis)]

In the present test, each of the subjects was made to ingest each test beverages, and their respiratory quotients and lipid oxidation rates were measured. The respiratory quotients were measured with the same method as in Test 1. Here, the lipid oxidation rate is the amount of fat consumed through energy metabolism, and determined, as with the case of respiratory quotients, on the basis of analysis results on expired breath obtained by using an Aero Monitor (R) (manufactured by MINATO

### MEDICAL SCIENCE CO., LTD.).

The present test was carried out as a single-ingestion crossover study for 34 adult males (BMI: 18.5 kg/m² or higher and lower than 25 kg/m²) as subjects. The specific testing method of the present test and the results of the test are shown in the following.

### (Testing Method)

In the present test, the test beverages of Comparative Example 3 and Example 5 used in Test 3 were used, and a test solution obtained by adding ethyl caproate into the test beverage (sweet-and-sour solution) of Comparative Example 3 to reach a concentration of 0.1 ppm was used as a test beverage of Example 10. The test was performed three times at intervals of 1 week or longer, and the test beverages were randomly ingested. The test beverages of Examples 5 and 10 respectively give 0.17 mg and 0.017 mg of ethyl caproate as the amount of ethyl caproate to be ingested. Ethanol was used as the solvent for ethyl caproate for the test beverages of Example 5 and Example 10, and a consistent ethanol concentration of 0.0999% was used for the test beverages in all cases.

Before performing the present test, the subjects were provided with identical meals for 3 days before the test, and forced to abstain from alcohol from the previous night. On the day of the test, the subjects were forbidden from ingesting any food or drink, except water. The subjects were each provided with the same clothes for the test.

The subjects were fasted for 12 hours from 21:00 on the previous day, and measurement of respiratory quotients was carried out in the same manner as in Test 1 under the conditions shown in Figure 1. Along with the measurement of respiratory quotients, measurement of lipid oxidation rates was carried out. The measurements of respiratory quotients and lipid oxidation rates were continuously carried out for 60 minutes. Thereafter, the same measurement was further performed twice at intervals of 1 week or longer. Beverages to be ingested were randomly assigned to every subject to make the subjects ingest each beverage once.

Data to be analyzed were as follows: in 20 minutes of resting in a sitting position from the initiation of measurement of respiratory quotients and lipid oxidation rates, respiratory quotients and lipid oxidation rates for 10 minutes from minute 11 to minute 20 were employed as respiratory quotients during resting and lipid oxidation rates during resting, and the averages of them were calculated.

### (Test Results)

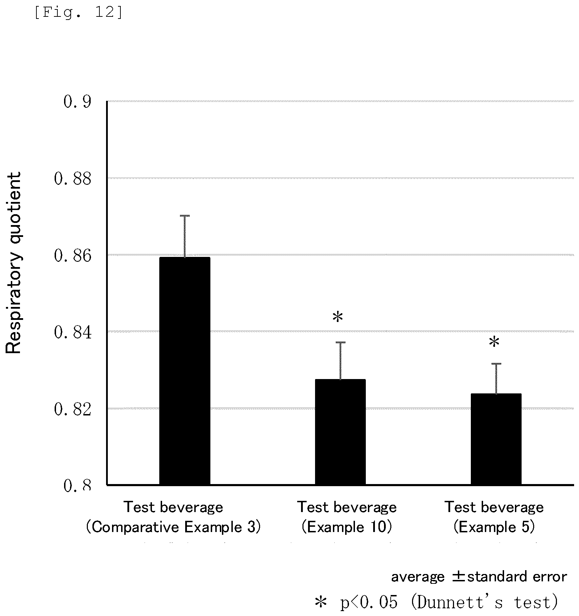

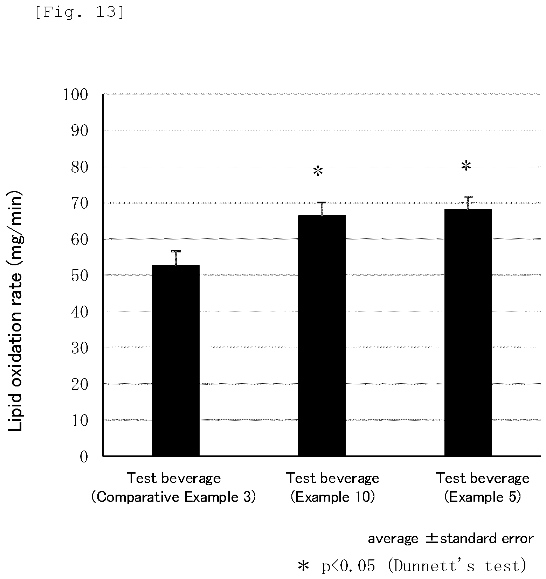

Figure 12 shows the results on respiratory quotients, and Figure 13 shows the results on lipid oxidation rates. In the present test, intergroup statistical significance test was carried out by Dunnett's test for the averages for the groups. Significance levels of p < 0.05 were each determined to be a significant difference. In the present test, data analysis was performed with Excel (manufactured by Microsoft Corporation).

As demonstrated in Figure 12, it was found that the group with ingestion of the test beverage of Example 10 exhibited significantly lower respiratory quotients during resting than the group with ingestion of the test beverage of Comparative Example 3 exhibited. It was also found that the group with ingestion of the test beverage of Example 5 exhibited significantly lower than the group with ingestion of the test beverage of Comparative Example 3 exhibited.

As demonstrated in Figure 13, it was found that the group with ingestion of the test beverage of Example 10 exhibited significantly higher lipid oxidation rates during resting than the group with ingestion of the test beverage of Comparative Example 3 exhibited. It was also found that the group with ingestion of the test beverage of Example 5 exhibited significantly higher values than the group with ingestion of the test beverage of Comparative Example 3 exhibited.

As described above, a reduced respiratory quotient indicates the increase in the amount of fat consumed, and an increased lipid oxidation rate indicates the increase in the amount of fat consumed through energy metabolism. Therefore, the results of the present test confirmed that the test beverages of Example 5 and Example 10 promote both lipolysis and fat combustion, and thus promote metabolism of fat.

### [Test 7 (Respiratory Quotient Measurement Test - Carbonated Water Basis)]

In the present test, each of the subjects was made to ingest each test beverage, and their respiratory quotients and lipid oxidation rates were measured. For measurement of respiratory quotients and lipid oxidation rates, an Aero Monitor (R) was used as in Test 6. The present test was carried out for four adult males as subjects. The specific testing method of the present test and the results of the test are shown in the following.

### (Testing Method)

In the present test, each subject was made to ingest different test beverages on day 1 and day 2 of test. As the test beverage on day 1 of test, 200 mL of carbonated water (Comparative Example 7) was used. As the test beverage on day 2 of test, 200 mL of a test solution obtained by adding ethyl caproate into carbonated water to reach a concentration of 1 ppm (Example 11) was used. The test beverage of Example 11 gives 0.17 mg of ethyl caproate as the amount of ethyl caproate to be ingested. "WILKINSON TANSAN"(manufactured by ASAHI SOFT DRINKS CO., LTD.) was used for the carbonated water. Ethanol was used as the solvent for ethyl caproate for the test beverage of Example 11, and a consistent ethanol concentration of 0.0999% was used for the test beverage in all cases.

Before performing the present test, the subjects were forced to abstain from alcohol from the night before the test. The subjects were fasted for 12 hours from 21:00 on the previous day, and measurement of respiratory quotients was carried out in the same manner as in Test 1 under the conditions shown in Figure 1. Along with the measurement of respiratory quotients, measurement of lipid oxidation rates was carried out. The measurements of respiratory quotients and lipid oxidation rates were carried out in a thermostatic/humidistatic experimental room set to a temperature of 23 ± 2°C and a humidity of 45 ± 5%, and those on day 1 and day 2 of test were carried out at the same time of day under the same conditions.

Data to be analyzed were as follows: in 20 minutes of resting in a sitting position from the initiation of measurement of respiratory quotients and lipid oxidation rates, respiratory quotients and lipid oxidation rates for 10 minutes from minute 11 to minute 20 were employed as respiratory quotients during resting and lipid oxidation rates during resting; in 30 minutes of exercise, respiratory quotients and lipid oxidation rates for 20 minutes from minute 11 to minute 30 were employed as respiratory quotients during exercising and lipid oxidation rates during exercising, respectively, and the averages of them were calculated.

### (Test Results)

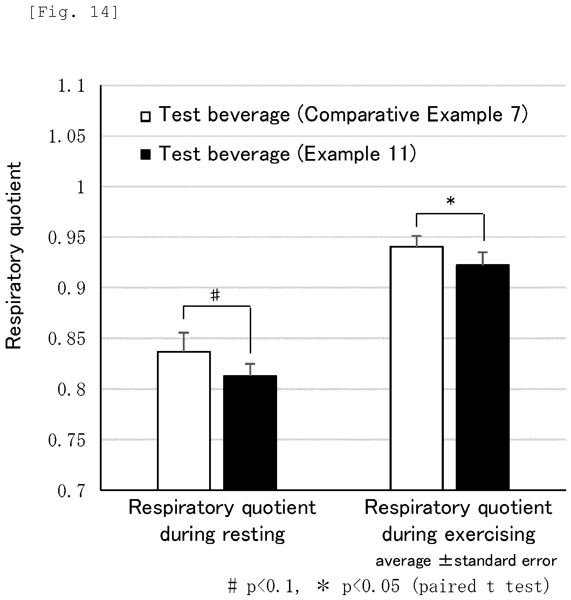

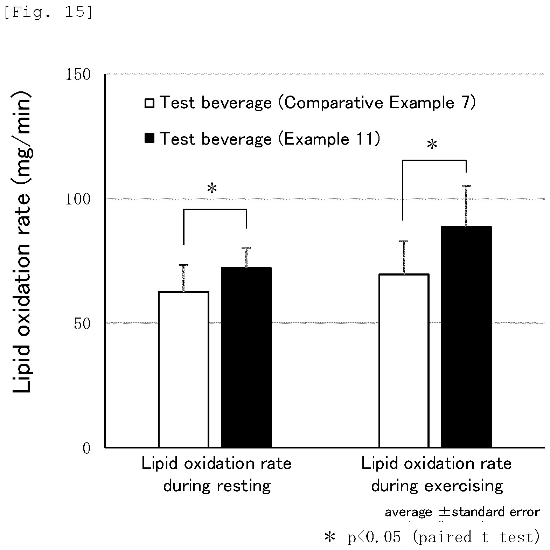

Figure 14 shows the results on respiratory quotients, and Figure 15 shows the results on lipid oxidation rates. In the present test, statistical significance test was carried out by paired t-test for the averages for the groups. Significance levels of p < 0.05 were each determined to be a significant difference, and significance levels of p < 0.1 were each determined to be a significant tendency. Data analysis was performed with Excel (manufactured by Microsoft Corporation).

As demonstrated in Figure 14, it was found that the group with ingestion of the test beverage of Example 11 exhibited a tendency of lower respiratory quotients during resting than the group with ingestion of the test beverage of Comparative Example 7 exhibited. It was also found that the group with ingestion of the test beverage of Example 11 exhibited significantly lower respiratory quotients during exercising than the group with ingestion of the test beverage of Comparative Example 7 exhibited.

As demonstrated in Figure 15, it was found that the group with ingestion of the test beverage of Example 11 exhibited significantly higher lipid oxidation rates during resting than the group with ingestion of the test beverage of Comparative Example 7 exhibited. It was also found that the group with ingestion of the test beverage of Example 11 exhibited significantly higher lipid oxidation rates during exercising than the group with ingestion of the test beverage of Comparative Example 7 exhibited.

As described above, a reduced respiratory quotient indicates the increase in the amount of fat consumed, and an increased lipid oxidation rate indicates the increase in the amount of fat consumed through energy metabolism. Therefore, the results of the present test confirmed that the test beverage of Example 11 promotes both lipolysis and fat combustion, and thus promotes metabolism of fat.

### [Test 8 (Body Temperature Measurement Test - Isoamyl Acetate)]

In the present test, each of the subjects was made to ingest each test beverage, and their interscapular skin temperature and tympanic temperature (hereinafter in Test 8, these are also referred to as "body temperature", collectively,) after the ingestion were measured. The present test was carried out as a single-ingestion crossover study for six adult males and females (two males, four females) as subjects. In the present test, interscapular skin temperature was measured with a myBeat (R) WHS-2 (manufactured by UNION TOOL CO.), and tympanic temperature was measured with the ear thermometer CE Thermo 2 (manufactured by NIPRO CORPORATION).

### (Testing Method)

In the present test, each subject was made to ingest different test beverages on day 1 and day 2 of test. As the test beverage on day 1 of test, 200 mL of water (Comparative Example 8) was used. As the test beverage on day 2 of test, 200 mL of a test solution obtained by adding isoamyl acetate into water to reach a concentration of 1 ppm (Example 12) was used. The test beverage of Example 12 gives 0.18 mg of isoamyl acetate as the amount of isoamyl acetate to be ingested. Ethanol was used as the solvent for isoamyl acetate for the test beverage of Example 12, and a consistent ethanol concentration of 0.0999% was used for the test beverage in all cases. "Isoamyl acetate ≥97%, FCC, Food Grade" (manufactured by Sigma-Aldrich Co. LLC) was used for the isoamyl acetate as a raw material of the test beverage. The log Pow of isoamyl acetate was 2.0 (calculated with the same method as described later in Test 9).

Before performing the present test, the subjects were forced to abstain from alcohol from the night before the test. On the day of the test, the subjects were forbidden from taking coffee or a highly spiced meal, made to finish lunch before 12:30, and forbidden from eating or drinking, except drinking water, after 12:30. The subjects were each made to wear the same specified short-sleeve shirt and long pants as clothes for the test.

Measurement of body temperature was carried out, in the same manner as in Test 2, under conditions shown in Figure 3, and the measurements on day 1 and day 2 of test were carried out at the same time of day under the same conditions.

With the measurements at the timing immediately after drinking a test beverage, 15:30, defined as initial values (0-min data), average ± standard error at each time was calculated for each group on the basis of measurements at 10-minute intervals from the timing immediately after drinking a test beverage to the completion of measurement at 16:30 (measurements at min 10, 20, 30, 40, 50, and 60).

### (Test Results)

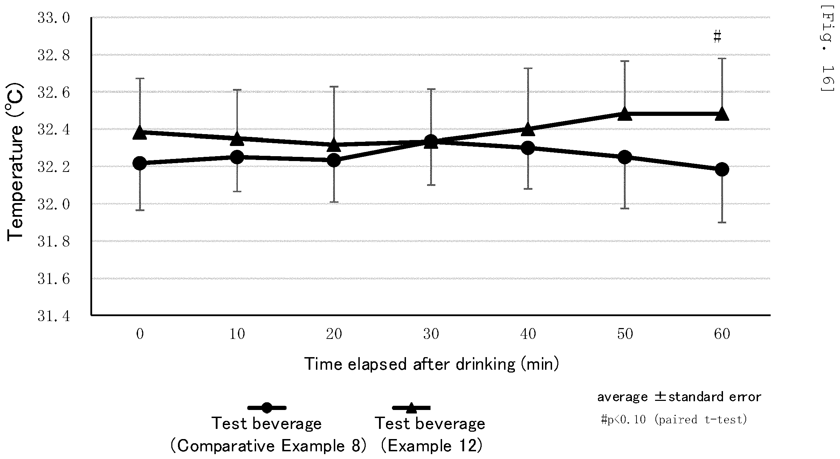

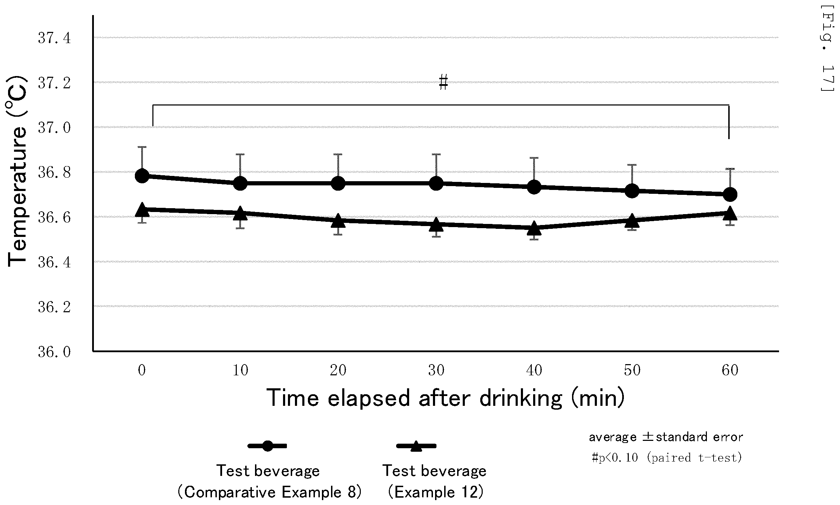

The results are shown in Figure 16 and Figure 17. Figure 16 shows the results on interscapular skin temperature, and Figure 17 shows the results on tympanic temperature. In the present test, statistical significance test was carried out by paired t-test for differences between the groups at identical times. Significance levels of p < 0.05 were each determined to be a significant difference, and significance levels of p < 0.10 were each determined to be a significant tendency. In the present test, data analysis was performed with Excel (manufactured by Microsoft Corporation).

As demonstrated in Figure 16, an increasing tendency in interscapular skin temperature was found for the group with ingestion of the test beverage of Example 12, in particular, from minute 30 after drinking. By contrast, such an increasing tendency was not found for the group with ingestion of the test beverage of Comparative Example 8.

As demonstrated in Figure 17, a continuous decreasing tendency in tympanic temperature was found for the group with ingestion of the test beverage of Comparative Example 8 after min 0, the timing immediately after drinking. By contrast, an increasing tendency appearing after min 40 after drinking was found for the group with ingestion of the test beverage of Example 12.

Statistical test by intergroup comparison found no significant difference in interscapular skin temperature shown in Figure 16 between the group with ingestion of the test beverage of Example 12 and the group with ingestion of the test beverage of Comparative Example 8 at min 0, the timing immediately after drinking. On the other hand, at min 60 after drinking, the group with ingestion of the test beverage of Example 12 exhibited higher interscapular skin temperature as a tendency than the group with ingestion of the test beverage of Comparative Example 8 exhibited.

Intragroup comparison of tympanic temperature shown in Figure 17 found that tympanic temperature at min 60 tended to be lower than that at min 0 in the group with ingestion of the test beverage of Comparative Example 8. By contrast, there was no such tendency in the group with ingestion of the test beverage of Example 12.

As described above, increase in interscapular skin temperature and reduction in lowering of tympanic temperature indicate promoted production of body heat. In particular, tympanic temperature is body temperature reflecting core body temperature, and hence reduction in lowering of tympanic temperature indicates that production of body heat is promoted even in the inside of a body. Because the interscapular part is in the vicinity of the brown adipose tissue, increase in interscapular skin temperature indicates that fatty acids are consumed in the brown adipose tissue and production of body heat is promoted. Therefore, the results of the present test confirmed that the test beverage of Example 12 promoted combustion of fat and further promoted production of body heat.

### [Test 9 (TRPA1 Activity Test)]

In the present test, test solutions of Examples 13 to 28 and Comparative Examples 9 to 14 described later were used in place of the test solutions used in First Testing Method of Test 5. Except that, the amount of change in RFU before and after addition of each test solution (ΔRFU) was determined for cells with forced expression of TRPA1 and non-transformed cells with the same method as First Testing Method of Test 5.

The test solutions of Examples 13 to 28 and Comparative Examples 10 to 14 used in the present test were each obtained by adding a compound listed in Table 7 into HEPES buffer. The octanol/water partition coefficient (log Pow) of each compound listed in Table 7 was calculated with XlogP (Shanghai Institute of Organic Chemistry, Chinese Academy of Sciences, http://WWW.sio-ccbg.ac.cn/software/xlogp3/), which is software using the XlogP3 algorithm. Each test solution was added as 50-µL portions to wells, and the concentrations of the compounds were set to concentrations shown in Table 7. HEPES buffer was used as the test solution of Comparative Example 9 for a negative control (N.C.), and added as 50-µL portions to wells.

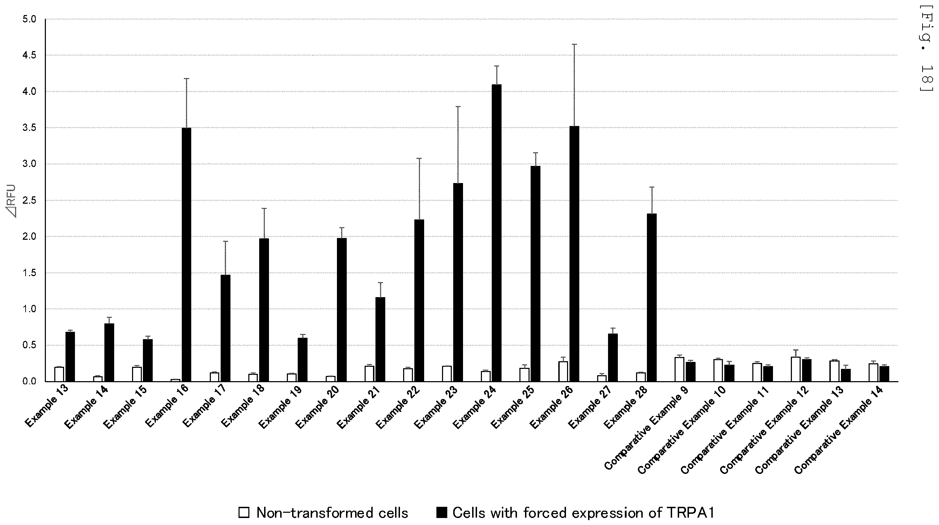

Table 7 and Figure 18 show average ± standard deviation of maximum values of ΔRFU. In the present test, SoftMax Pro Vr. 6 (manufactured by Molecular Devices, LLC.) and Excel (manufactured by Microsoft Corporation) were used for data analysis.

As demonstrated in Table 7 and Figure 18, the test solutions of Comparative Examples 9 to 14 did not provide maximum values of ΔRFU in cells with forced expression of TRPA1 higher than maximum values of ΔRFU in non-transformed cells, and thus the response of cells with forced expression of TRPA1 was not found. On the other hand, the test solutions of Examples 13 to 28 provided maximum values of ΔRFU in cells with forced expression of TRPA1 higher (specifically, 2.9 or more times higher) than maximum values of ΔRFU in non-transformed cells, and thus the cellular response of cells with forced expression of TRPA1 was successfully found. These results confirmed that the test solutions of Examples 13 to 28 activate TRPA1.

**[Table 7]**

| | Compound name | Number of carbon atoms of R1 | Number of carbon atoms of R2 | logPow | Concentration after adding (ppm) | Δ RFU in non-transformed cells | Δ RFU in cells with forced expression of TRPA1 | Δ RFU in non-transformed cells / Δ RFU in cells with forced expression of TRPA1 |
|---|---|---|---|---|---|---|---|---|
| Example 13 | Isoamyl acetate | 1 | 5 | 2.0 | 16.6 | 0.2 ± 0.0 | 0.7 ± 0.0 | 3.4 |
| Example 14 | Pentyl acetate | 1 | 5 | 1.9 | 14.8 | 0.1 ± 0.0 | 0.8 ± 0.1 | 12.1 |
| Example 15 | 2-Methylbutyl acetate | 1 | 5 | 2.1 | 14.8 | 0.2 ± 0.0 | 0.6 ± 0.0 | 2.9 |
| Example 16 | Heptyl acetate | 1 | 7 | 2.9 | 18.2 | 0.0 ± 0.0 | 3.5 ± 0.7 | 127.8 |
| Example 17 | Isoamyl propionate | 2 | 5 | 2.5 | 18.2 | 0.1 ± 0.0 | 1.5 ± 0.5 | 12.4 |
| Example 18 | Isoamyl butyrate | 3 | 5 | 2.6 | 21.7 | 0.1 ± 0.0 | 2.0 ± 0.4 | 19.3 |
| Example 19 | Ethyl valerate | 4 | 2 | 1.9 | 14.8 | 0.1 ± 0.0 | 0.6 ± 0.1 | 5.6 |
| Example 20 | Ethyl 4-methylvalerate | 5 | 2 | 2.1 | 15.0 | 0.1 ± 0.0 | 2.0 ± 0.1 | 27.6 |
| Example 21 | Methyl caproate | 5 | 1 | 2.5 | 14.6 | 0.2 ± 0.0 | 1.2 ± 0.2 | 5.4 |
| Example 22 | Ethyl caproate | 5 | 2 | 2.4 | 16.6 | 0.2 ± 0.0 | 2.2 ± 0.8 | 12.7 |
| Example 23 | Propyl caproate | 5 | 3 | 2.9 | 18.2 | 0.2 ± 0.0 | 2.7 ± 1.1 | 12.9 |
| Example 24 | Butyl caproate | 5 | 4 | 3.3 | 19.8 | 0.1 ± 0.0 | 4.1 ± 0.3 | 29.1 |
| Example 25 | Isoamyl caproate | 5 | 5 | 3.7 | 24.9 | 0.2 ± 0.0 | 3.0 ± 0.2 | 16.2 |
| Example 26 | Pentyl caproate | 5 | 5 | 3.8 | 21.4 | 0.3 ± 0.1 | 3.5 ± 1.1 | 12.8 |
| Example 27 | Methyl caprylate | 7 | 1 | 3.6 | 18.0 | 0.1 ± 0.0 | 0.7 ± 0.1 | 8.3 |
| Example 28 | Ethyl caprylate | 7 | 2 | 3.5 | 19.8 | 0.1 ± 0.0 | 2.3 ± 0.4 | 19.0 |
| Comparative Example 9 | N.C. | | | | | 0.3 ± 0.0 | 0.3 ± 0.0 | 0.8 |
| Comparative Example 10 | Ethyl acetate | 1 | 2 | 0.7 | 10.2 | 0.3 ± 0.0 | 0.2 ± 0.1 | 0.7 |
| Comparative Example 11 | Propyl acetate | 1 | 3 | 1.2 | 11.5 | 0.3 ± 0.0 | 0.2 ± 0.0 | 0.8 |
| Comparative Example 12 | Ethyl propionate | 2 | 2 | 1.2 | 11.5 | 0.3 ± 0.1 | 0.3 ± 0.0 | 0.9 |
| Comparative Example 13 | Isoamyl caprylate | 9 | 5 | 5.9 | 28.2 | 0.3 ± 0.0 | 0.2 ± 0.1 | 0.6 |
| Comparative Example 14 | Ethyl laurate | 11 | 2 | 5.6 | 26.6 | 0.2 ± 0.0 | 0.2 ± 0.0 | 0.8 |

The results of Tests 1 to 9 described above confirmed that lipid metabolism including lipolysis and fat combustion can be promoted with compound (1) having an octanol/water partition coefficient (log Pow) in the range of 1.9 or more and 3.8 or less. In addition, it was confirmed that generation of body heat is promoted as a result of promoted fat combustion. Although why lipid metabolism including lipolysis and fat combustion is promoted has not been precisely clarified, it was inferred to be one reason that TRPA1 is activated by compound (1) having an octanol/water partition coefficient (log Pow) in the range of 1.9 or more and 3.8 or less, promoting lipid metabolism via neurotransmission.

## Claims

1. A lipid metabolism promoter comprising a compound represented by general formula (1): 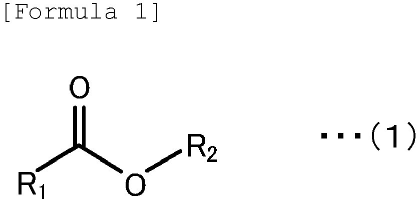 wherein R₁ and R₂ each independently represent an alkyl group having one, two, three, four, five, six, or seven carbon atoms,
as an active ingredient, wherein
the compound represented by general formula (1) has a log Pow indicating an octanol/water partition coefficient of 1.9 or more and 3.8 or less.

2. The lipid metabolism promoter according to claim 1, wherein R₁ is one group selected from the group consisting of a methyl group, an ethyl group, a n-propyl group, a n-butyl group, a n-pentyl group, an isopentyl group, a n-hexyl group, and a n-heptyl group.

3. The lipid metabolism promoter according to claim 1 or 2, wherein R₂ is one group selected from the group consisting of a methyl group, an ethyl group, a n-propyl group, a n-butyl group, a n-pentyl group, an isopentyl group, a 2-methylbutyl group, a n-hexyl group, and a n-heptyl group.

4. The lipid metabolism promoter according to any one of claims 1 to 3, wherein the compound represented by general formula (1) is at least one compound selected from the group consisting of ethyl caproate, methyl caproate, propyl caproate, butyl caproate, pentyl caproate, ethyl caprylate, isoamyl acetate, isoamyl propionate, isoamyl butyrate, isoamyl caproate, pentyl acetate, heptyl acetate, methyl caprylate, ethyl 4-methylvalerate, 2-methylbutyl acetate, and ethyl valerate.

5. The lipid metabolism promoter according to any one of claims 1 to 4, wherein the amount of the compound represented by general formula (1) to be ingested by an adult per day is 0.017 mg or more and 1.8 mg or less.

6. The lipid metabolism promoter according to any one of claims 1 to 5, wherein the lipid metabolism promoter is a lipolysis promoter.

7. The lipid metabolism promoter according to any one of claims 1 to 5, wherein the lipid metabolism promoter is a fat combustion promoter.

8. A body heat production promoter comprising a compound represented by general formula (1): 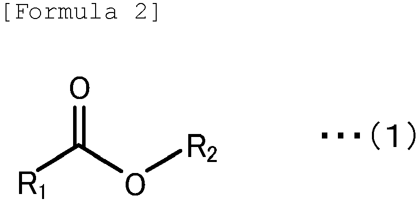 wherein R₁ and R₂ each independently represent an alkyl group having one, two, three, four, five, six, or seven carbon atoms,
as an active ingredient, wherein
the compound represented by general formula (1) has a log Pow indicating an octanol/water partition coefficient of 1.9 or more and 3.8 or less.
